# EUROPEAN PATENT SPECIFICATION

(11) **EP 0 339 842 B1**
(45) Date of publication and mention of the grant of the patent: **06.11.1996**
(21) Application number: 89303700.2
(22) Date of filing: 13.04.1989
(51) Int. Cl.: C07H 21/00, C07H 19/067, C07H 19/167, A61K 31/70, C07J 51/00, A61K 31/58

(54) **Novel oligoribonucleotide derivatives and application thereof to antiviral agents**
Oligoribonukleotid-Derivate und ihre Anwendung als antivirale Mittel
Dérivés d'oligoribonucléotides et leur utilisation comme agents antiviraux

(30) Priority: 27.04.1988 JP 104943/88; 06.06.1988 JP 138966/88; 06.07.1988 JP 168142/88; 12.09.1988 JP 227887/88; 22.09.1988 JP 238481/88; 02.02.1989 JP 24372/89; 16.03.1989 JP 64058/89
(43) Date of publication of application: 02.11.1989
(60) Divisional application: 96106543.0
(73) Proprietor: ISIS PHARMACEUTICALS, INC., Carlsbad, CA 92008 (US)
(72) Inventor: Shibahara, Susumu Central Research Laboratories, Kawasaki-ku Kawasaki-shi Kanagawa-ken (JP); Morisawa, Hirokazu Central Research Laboratories, Kawasaki-ku Kawasaki-shi Kanagawa-ken (JP); Nakajima, Hideki, Ube-shi Yamaguchi-ken (JP); Yamamoto, Naoki, Ube-shi Yamaguchi-ken (JP); Mukai, Sachiko c/o Central Research Laboratories, Kawasaki-ku Kawasaki-shi Kanagawa-ken (JP)
(74) Representative: Bond, Bentley George

(56) References cited:
- EP-A- 0 169 775
- VIROLOGY vol. 42, no. 2 , October 1970 , ORLANDO, FL, USA pages 421 - 428 E. DE CLERCO ET AL 'The Anti-Viral Activity of Thiophosphate-Substituted Polyribonucleotides "In Vitro" and "In Vivo"'
- CHEMICAL ABSTRACTS, vol. 106, no. 19, 11 May 1987, Columbus, Ohio, US; abstract no. 156829, E. OTSUKA ET AL '2'-O-Methyl RNA' page 726 ;column 2 ;
- NATURE vol. 313, no. 6000 , January 1985 , LONDON GB pages 277 - 284 L. RATNER ET AL 'Complete Nucleotide Sequence of the AIDS Virus, HTLV-III'
- BIOCHEMISTRY. vol. 18, no. 19 , 18 September 1979 , EASTON, PA US pages 4116 - 4120 D. YEE ET AL 'Mechanistic Studies on Deoxyribonucleic Acid Dependent Ribonucleic Acid Polymerase from Escherichia coli Using Phosphorothioate Analogues. 1. Initiation and Pyrophosphate Exchange Reactions'
- BIOCHEMISTRY. vol. 21, no. 23 , 9 November 1982 , EASTON, PA US pages 5877 - 5885 F. R. BRYANT ET AL 'Phosphorothioate Substrates for T4 RNA Ligase'
- NUCLEIC ACIDS RESEARCH. vol. 16, no. 4 , 25 February 1988 , ARLINGTON, VIRGINIA US pages 1577 - 1591 A. W. NICHOLSON ET AL 'Accurate "In Vivo" Cleavage by RNAse III of Phosphorothioate-Substituted RNA Processing Signals in Bacteriophage T7 early mRNA'
- JOURNAL OF THE AMERICAN CHEMICAL SOCIETY. vol. 103, no. 3 , 11 February 1981 , GASTON, PA US pages 696 - 697 F. R. BRYANT ET AL 'On the Mechanism of T4 RNA Ligase'
- NUCLEIC ACIDS RESEARCH SYMPOSIUM SERIES - SEVENTH SYMPOSIUM ON THE CHEMISTRY OF NUCLEIC ACID COMPONENTS HELD AT BECHYNE CASTLE, CZECHOSLOVAKIA, FROM 30-08-87 - 05-09-87 no. 18 , 1987 , IRL PRESS LTD, OXFORD GB pages 277 - 280 C. DEENEY ET AL 'Self Splicing of Tetrahymena rRNA Can Proceed with Phosphorothioate Substituent at the Splice Sites'
- PROCEEDINGS OF THE NATIONAL ACADEMY OF SCIENCES OF USA vol. 84, no. 21 , November 1987 , WASHINGTON US pages 7706 - 7710 M. MATSUKURA ET AL 'Phosphorothioate Analogs of Oligodeoxynucleotides: Inhibitors of Replication and Cytopathic Effects of Human Immunodeficiency Virus'
- NUCLEIC ACIDS RESEARCH. vol. 17, no. 1 , 11 January 1989 , ARLINGTON, VIRGINIA US pages 239 - 252 S. SHIBAHARA ET AL 'Inhibition of Human Immunodeficiency Virus (HIV-1) Replication by Synthetic Oligo-RNA Derivatives'

## Description

The present invention relates to novel compounds, namely 2′-O-methylribooligonucleotide phosphorothioate derivatives, 2′-O-methylribooligonucleotide derives and oligoribonucleotide derivatives, which can inhibit proliferation of AIDS virus (HIV-1, HIV-2, etc.) causing AIDS (acquired immunodeficiency syndrome), to make AIDS virus avirulent, novel intermediates for producing the same and the application of these derivatives to drugs, for example, antiviral agents such as anti-AIDS drugs or the like.

AIDS (acquired immunodeficiency syndrome) was found in 1981 and it has been clarified that the cause lies in HIV (human immunodeficiency virus, hereafter simply referred to as AIDS virus) belonging to the family retro virus. The number of patients shows a yearly increase all over the world. According to the WHO's report, the number of patients exceeded 110,000 in the world as of August 31, 1988. In Japan, 90 patients are reported (as of August 31, 1988). Furthermore, if carriers infected with the virus but developing no AIDS yet are included, the number of patients will be more than 10,000,000 (estimated by WHO) in the world and 1048 in Japan (reported by the Ministry of Health and Welfare, as of August 31, 1988). A drug for completely curing AIDS that is a serious disease has not been found so far. 3′-Deoxy-3′-azidothymidine (hereafter simply referred to as "AZT") has been used in the clinical field as a drug for retarding the progress of AIDS and improving the clinical syndrome of the patient. However, side effect such as bone marrow impairment, etc. associated with its effectiveness are reported (cf. The New England Journal of Medicine, 317, 192-197, 1987). The mechanism of AZT is based on inhibition of the activity of reverse transcriptase possessed by the retro virus itself which is utilized, after invasion of the virus into host cells, to transcribe its genomic RNA onto DNA (Mitsuya, H., et al., Proc. Natl. Acad. Sci. USA, 82, 7096-7100, 1985) and it is thus probable that the mechanism would be related to the side effects.

On the other hand, it has been recently reported that phosphorothioate derivatives of deoxyoligonucelotides exhibit an anti-AIDS viral activity (cf. Proc. Natl. Acad. Sci. USA, 84, 7706-7710, 1987).

In Erik De Clercq et al., Virology, 42, 421-428 (1970) "The Antiviral Activity of Thiophosphate-Substituted Polyribonucleotides in vitro and in vivo" there is described substitution of thiophosphate for phosphate in a polyribonucleotide. This resulted in an increase of its ability to induce in vitro resistance to virus infection, and showed an increased resistance to degradation by nucleases present in foetal calf serum.

In Otsuka and Inoue, Chem. Abstr., 106, 156829v (1987) "2'-O-Methyl RNA" there is disclosed 2'-O-methylnucleotides which were found to be resistant to nuclease, phosphodiesterase and phosphorylase, and were indicated to be useful as RNA and DNA probes.

In Ratner et al., Nature, 313, 277-284 (1985) "Complete Nucleotide Sequence of the AIDS virus, HTLV-III" the complete nucleotide sequence of two human T-cell leukaemia type III (HTLV-III) proviral DNAs is provided.

In Shibahara et al., Nucleic Acids Research, 17, 239-252 (1989) "Inhibition of Human Immunodeficiency Virus (HIV-1) Replication by Synthetic Oligo-RNA Derivatives" several synthetic 2'-O-methyl RNA oligomers and their derivatives were evaluated for inhibitory effects against HIV induced cytopathic effects.

The AIDS virus has extremely frequent variations among its strains, in respect of its envelope protein. Therefore, it is extremely difficult to develop an effective vaccine and chemotherapeutic agents as described above are desired. However, nucleocido derivatives such as AZT conventionally used as chemotherapeutic agents exhibit anti-AIDS viral activity on one hand but on the other hand, involve serious side effects. Accordingly, it has been desired to develop drugs having minimized side effects and high practicability. For achieving the purpose, deoxyoligonucleotide phosphorothioate derivatives are at the stage of basic studies but it is desired to develop drugs having a more potent activity and provide such drugs at low costs.

As a result of extensive investigations to solve the foregoing problems, the present inventors have found that newly produced 2'-O-methyloligoribonucleotide derivatives and oligoribonucleotide derivatives could inhibit infection and proliferation of the AIDS virus to and in host cells and are applicable as drugs for treating AIDS, and have accomplished the present invention based on this finding.

Thus, the present invention is directed to:
novel oligoribonucleotide derivatives represented by general formula (1) described below, and anti-AIDS viral compositions comprising the oligoribonucleotide derivatives as the effective ingredient;
novel 2'-O-methyloligoribonucleotide phosphorothioate derivatives containing a sequence complementary to gene RNA of the AIDS virus (HIV-1, HIV-2, etc. or DNA integrated into chromosome and represented by general formula (2) described below and anti-AIDS viral compositions comprising the derivatives as the effective ingredient;
novel 2'-O-methyloligoribonucleotide derivatives represented by general formula (3) described below and anti-AIDS viral compositions comprising the derivatives as the effective ingredient;
novel oligoribonucleotide derivatives represented by general formula (4) described below and anti-AIDS viral compositions comprising the derivatives as the effective ingredient.

Novel 2'-O-methyloligoribonucleotide derivatives represented by general formula (5) below and novel nucleoside derivatives represented by general formula (6) below are also described. 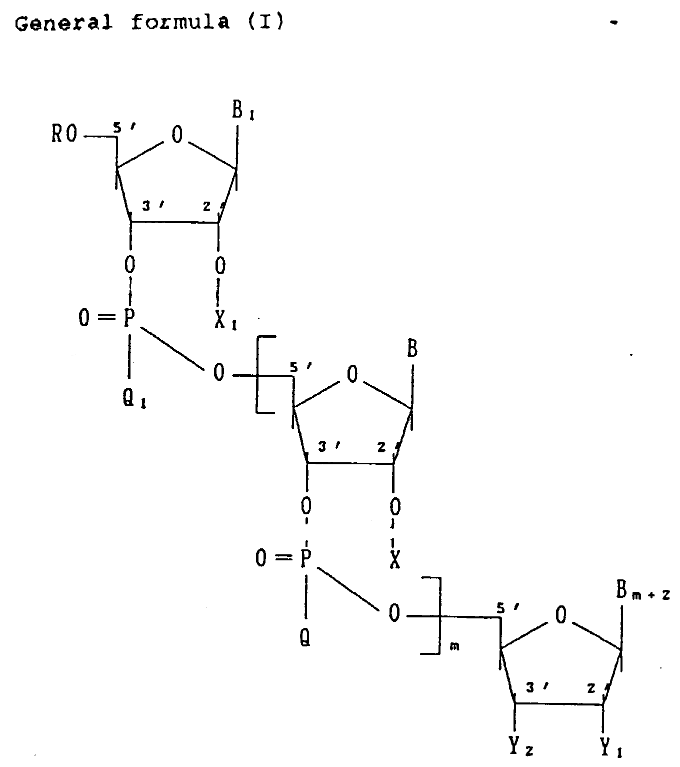 wherein:
m is an integer of 1 to 100;
B represents B₂, B₃, ..... B₍ₘ₊₁₎ and, B₁ through B₍ₘ₊₂₎, which may be the same or different, each represents any one of hypoxanthine-9-yl, guanine-9-yl, adenine-9-yl, cytosine-1-yl, uracil-1-yl and thymine-1-yl;
Q represents Q₂, Q₃, ..... Q₍ₘ₊₁₎ and, Q₁ through Q₍ₘ₊₁₎, independently represents any one of a thio anion, an oxo anion, an alkyl group, an aryloxy group, an alkylamino group, an arylamino group, an alkylthio group and an arylthio group (provided that at least one of Q₁ to Q₍ₘ₊₁₎ represents a thio anion);
X represents X₂, X₃, ..... X₍ₘ₊₁₎ and, X₁ through X₍ₘ₊₁₎, which may be the same or different, each represents any one of a hydrogen atom, methyl group or an acyl group having 1 to 20 carbon atoms (provided that all of them do not represent a hydrogen atom);
R represents a hydrogen atom, an acyl group having 1 to 20 carbon atoms, a phosphoryl group which may optionally have a substituent, a thiophosphoryl group which may optionally have a substituent or an alkylaminophosphoryl group which may optionally have a substituent; and
Y₁ and Y₂ independently represents methoxy group, an acyloxy group having 1 to 20 carbon atoms, a hydrogen atom, hydroxyl group, an alkyloxy group which may optionally have a substituent, a phosphoryloxy group which may optionally have a substituent, a thiophosphoryloxy group which may optionally have a substituent and an alkylaminophosphoryloxy group which may optionally have a substituent. Herein the alkyl represents a straight or branched chain hydrocarbon residue having 1 to 30 carbon atoms and the aryl represents a hydrocarbon residue having 6 to 30 carbon atoms including phenyl group.

In formula (1) described above, X is bound to an oxygen atom at the 2'-position in the sugar moiety, and the phosphorus atom (P) is bound to an oxygen atom at the 3'-position.

Further in formula (1) described above, examples of the substituent on R, Y₁ and Y₂ include cyanoethyl group, chlorophenyl group, monophosphoryl group, pyrophosphoryl group, a hydrocarbon residue having 1 to 20 carbon atoms, cholesteryl group and a group shown by J-(K-O-L)̵_{E}, which may be the same or different: provided that J represents: 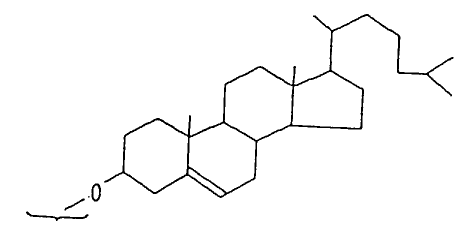 hydroxyl group or any one of the oligoribonucleotidyl group defined for the structural formula of said derivative according to claim 1, from which any one of RO, Y₁ and Y₂ is removed;
K represents any one of phosphoryl group, thiophosphoryl group and an alkylaminophosphoryl group having 1 to 10 carbon atoms;
L represents a hydrocarbon residue having 1 to 20 carbon atoms; and,
E represents an integer of 1 to 10.

The nucleotide sequence may not necessarily be complementary to the sequence of genomic RNA of AIDS virus or DNA integrated into chromosome.

In the formula described above, B₁ through B₍ₘ₊₂₎, Q₁ through Q₍ₘ₊₁₎ and X₁ through X₍ₘ₊₁₎ are shown from the 5′-end in the order of 1, 2, 3, 4, ... (m+1). For example, when m is 2, B₁ to B₍ₘ₊₂₎ are, in the order from the 5′-end, B₁, B₂, B₃, B₄; Q₁ to Q₍ₘ₊₁₎ are, in the order from the 5′-end, Q₁, Q₂, Q₃; and X₁ through X₍ₘ₊₁₎ are, in the order from the 5′-end, X₁, X₂, X₃; and the following structural formula is presented:

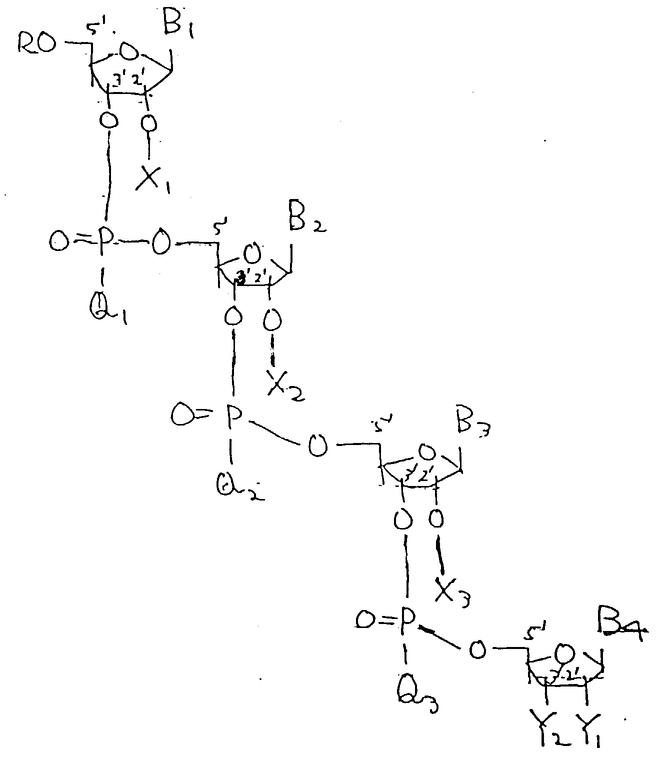

Likewise, when m is 100, B₁ to B₍ₘ₊₂₎ are, in the order from the 5'-end, B₁, B₂, B₃, ... B₁₀₀, B₁₀₁, B₁₀₂; Q₁ to Q₍ₘ₊₁₎ are, in the order from the 5'-end, Q₁, Q₂, Q₃, ... Q₁₀₀, Q₁₀₁; and X₁ through X₍ₘ₊₁₎ are, in the order from the 5'-end, X₁, X₂, X₃ ... X₁₀₀, X₁₀₁; and the following structural formula is presented: 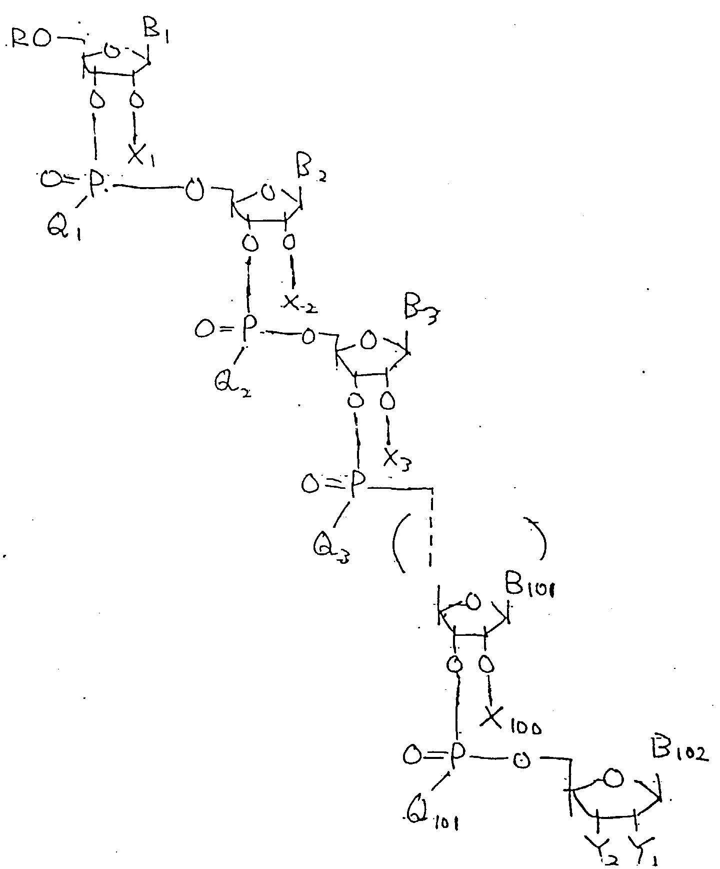 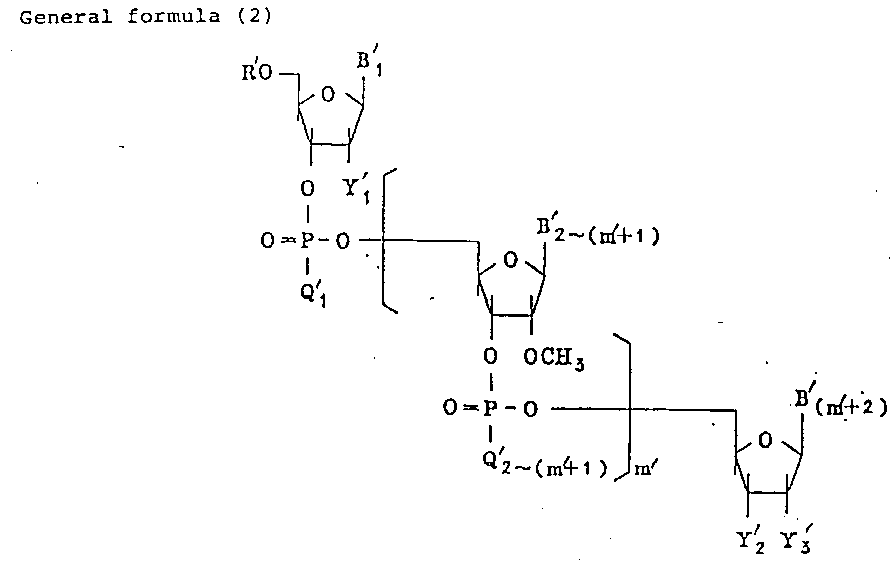 wherein:
m′ is an integer of 1 to 50;
B′₁ through B′_{(m′+2)}, which may be the same or different, each represents any one of adenine-9-yl (A), guanine-9-yl (G), cytosine-1-yl (C), uracil-1-yl (U), thymine-1-yl (T) and hypoxanthine-9-yl (H);
Q′₁ represents any one of a thio anion (S ⁻ ), an oxo anion (0 ⁻ ), an alkyl group, an alkyloxy group, an aryloxy group, an alkylamino group, an arylamino group, an alkylthio group and an arylthio group;
at least one of Q′₂ through Q′_{(m′+1)} represents a thio anion (S ⁻ ) and the balance is either a thio anion or an oxo anion (O ⁻ );
Y′₁ through Y′₃ independently represents methoxy group, an alkyloxy group which may optionally have a substituent, hydroxyl group, an alkylsilyl group and a hydrogen atom;
R′ represents a hydrogen atom, a phosphoryl group or thiophosphoryl group which may optionally have a substituent. Herein the alkyl represents a straight or branched chain hydrocarbon residue having 1 to 30 carbon atoms and the aryl represents a hydrocarbon residue having 6 to 30 carbon atoms including phenyl group. Examples of the substituent include cyanoethyl group, chlorophenyl group and a hydrocarbon residue having 1 to 10 carbon atoms.

In the formula described above, B′ and Q′ are shown in the order of 1, 2, 3, 4, ... (m′+2). For example, when m′ is 2, B′₂ to B'_{(m'+1)} are, in the order from the 5'-end, B'₂, B'₃; Q'₂ to Q'_{(m'+1)} are, in the order from the 5'-end, Q'₂, Q'₃; and the following structural formula is presented:

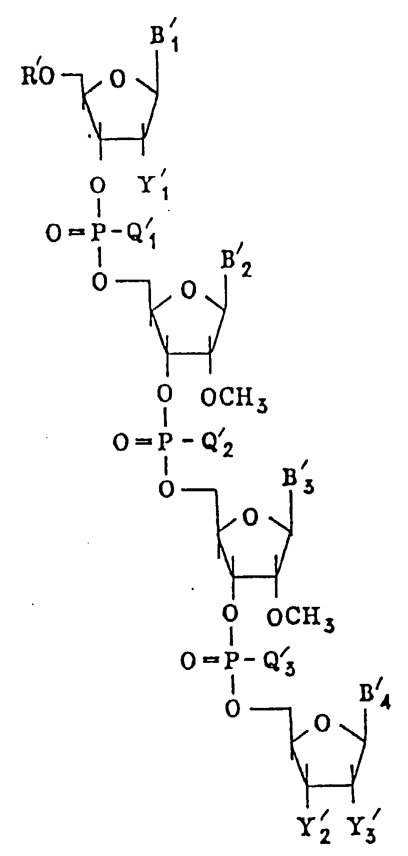

Likewise, when m' is 50, B'₂ to B'_{(m'+1)} are, in the order from the 5'-end, B'₂, B'₃, B'₄, ... B'₅₀, B'₅₁; Q'₂ to Q'_{(m'+1)} are, in the order from the 5'-end, Q'₂, Q'₃, Q'₄, ... Q'₅₀, Q'₅₁; and the following structural formula is presented: 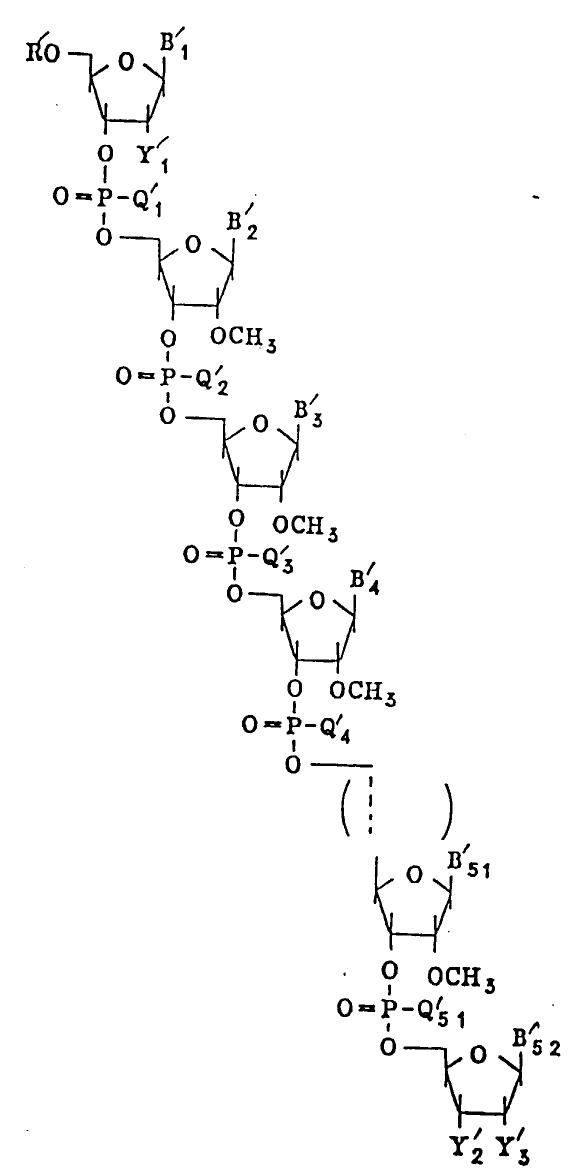 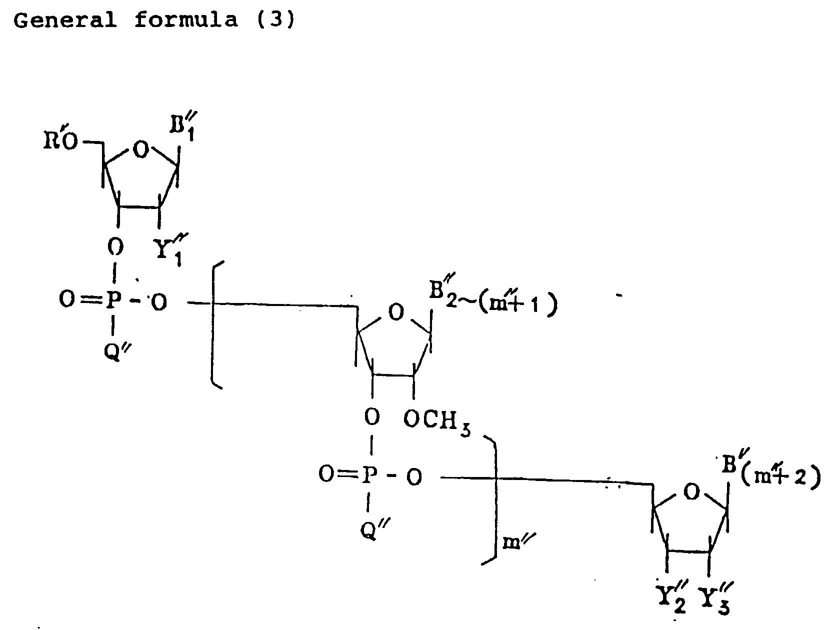 wherein:
m" is an integer of 1 to 50;
B"₁ through B"_{(m"+2)}, which may be the same or different, each represents any one of adenine-9-yl (A), guanine-9-yl (G), cytosine-1-yl (C), uracil-1-yl (U), thymine-1-yl (T) and hypoxanthine-9-yl (H);
Q" contains at least one thio anion and represents any one of a thio anion (S ^{⊖} ), an oxo anion (O^{⊖} ), an alkyl group, an alkyloxy group, an aryloxy group, an alkylamino group, an arylamino group, an alkylthio group and an arylthio group;
Y"₁ through Y"₃ independently represents methoxy group, an alkyloxy group which may optionally have a substituent, hydroxyl group, an alkylsilyl group and a hydrogen atom;
R" represents a hydrogen atom or a phosphoryl group which may optionally have a substituent.

Herein the alkyl represents a straight or branched chain hydrocarbon residue having 1 to 30 carbon atoms and the aryl represents a hydrocarbon residue having 6 to 30 carbon atoms including phenyl group. Examples of the substituent include cyanoethyl group, chlorophenyl group and a hydrocarbon residue having 1 to 10 carbon atoms. The nucleotide sequence may not necessarily be complementary to the sequence of genomic RNA of AIDS virus or DNA integrated into chromosome. 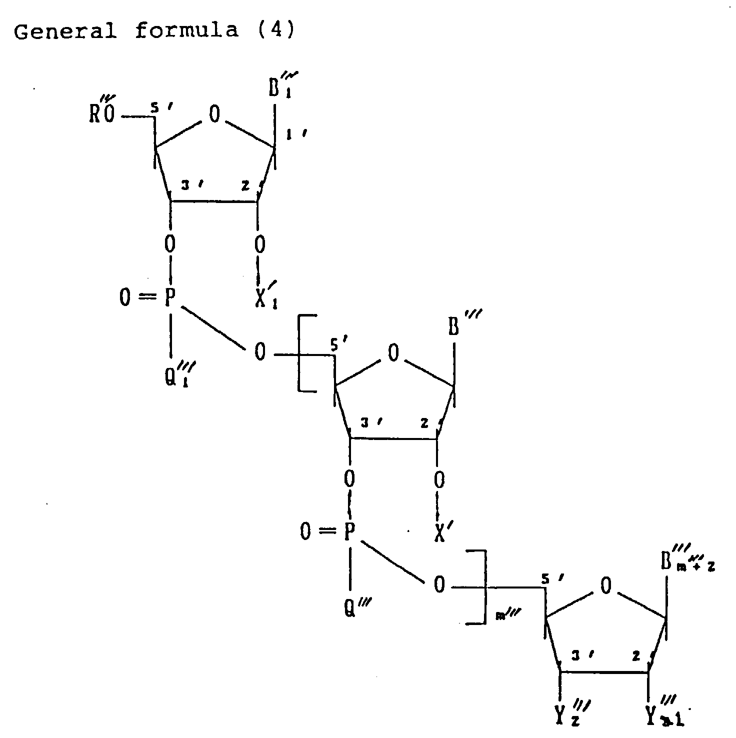 wherein:
m"'is an integer of 1 to 100;
B"' represents B"'₂, B"'₃, ..... B"'_{(m"' +1)}, provided that all of them do not represent adenine-9-yl; and, B"'₁ through B"'_{(m"'+2)}, which may be the same or different; each represents any one of hypoxanthine-9-yl, guanine-9-yl, adenine-9-yl, cytosine-1-yl, uracil-1-yl and thymine-1-yl;
Q"' represents Q"'₂, Q"'₃, ..... Q"'_{(m"'+1)} and, at least one of Q"'₁ to Q"'_{(m"'+1)} represents a thio anion. Q"'₁ through Q"'_{(m"'+1)}, independently represents any one of a thio anion, an oxo anion, an aryloxy group, an alkylamino group, an arylamino group, an alkylthio group and an arylthio group;
X' represents X'₂, X'₃, ..... X'_{(m"'+1)} and, X'₁ through X'_{(m"'+1)} each represents a hydrogen atom or methyl group (provided that all of them do not represent a methyl group and provided that all of them do not represent a hydrogen atom);
R"' represents any one of a hydrogen atom, an acyl group having 1 to 20 carbon atoms and a phosphoryl group which may optionally have a substituent;
Y"'₁ and Y"'₂ independently represents any one of methoxy group, an acyloxy group having 1 to 20 carbon atoms, an alkyloxy group which may optionally have a substituent, hydroxyl group, an alkylsilyl group, a hydrogen atom and a phosphoryl group which may optionally have a substituent.

Herein the alkyl represents a straight or branched chain hydrocarbon residue having 1 to 30 carbon atoms and the aryl represents a hydrocarbon residue having 6 to 30 carbon atoms including phenyl group. Examples of the substituent include cyanoethyl group, chlorophenyl group and a hydrocarbon residue having 1 to 20 carbon atoms.

In the formula described above, the oxygen atoms at the 2'-position and at the 3'-position in the sugar moiety of nucleoside monomer are bound to X' and to the phosphorus atom (P). The binding mode of the monomer at the sugar moiety is a 3'-5'bond.

The nucleotide sequence may not necessarily be complementary to the sequence of genomic RNA of AIDS virus or DNA integrated into chromosome.

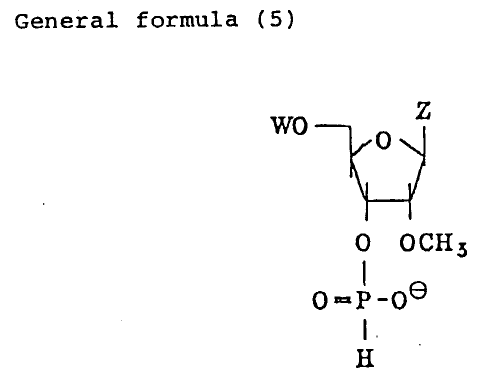

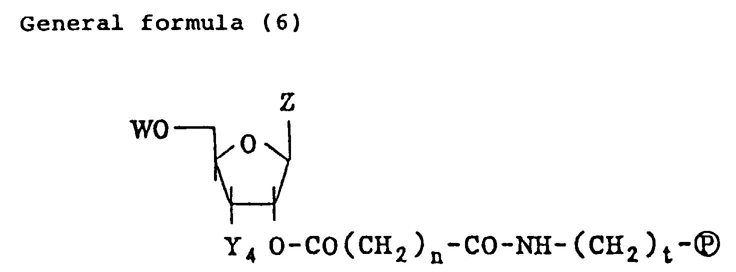

In the general formulae (5) and 6), W represents monomethoxytrityl group or dimethoxytrityl group; Y₄ represents methoxy group, an alkyloxy group which may optionally have a substituent or a hydrogen atom; n and t, which may be different from each other, or independently, each represents an integer of 1 to 30; Ⓟ represents any one of a controlled pore glass derivative, a polystyrene derivative or a silica gel derivative; and Z represents a substituent represented by any one of general formulae below:

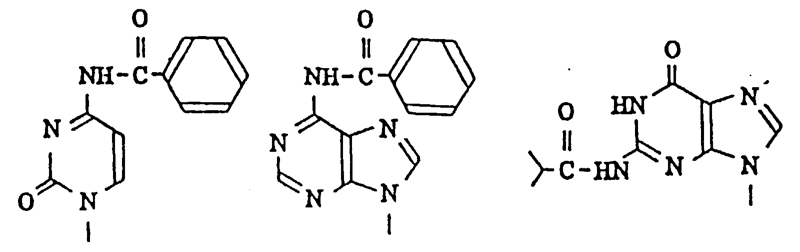

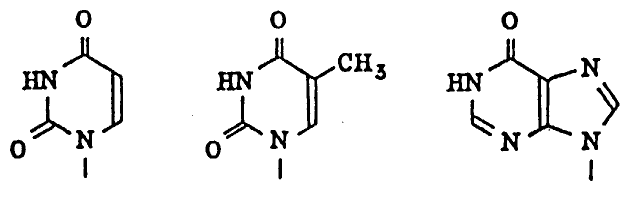

The carbon atom number of the alkyl moiety in the alkyloxy group is 1 to 10. Examples of the alkyloxy group having a substituent include: 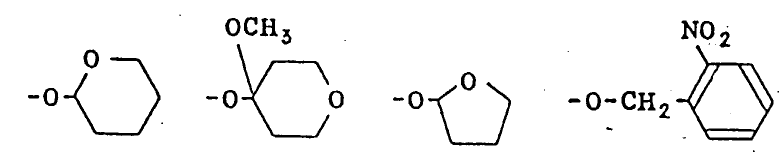 etc.

With respect to the antiviral activity of oligodeoxynucleotide, an example of using oligodeoxynucleotide for inhibiting proliferation of Raus sarcoma virus without converting it into any derivative is reported (Zamecnik, P.C., Proc. Natl. Acad. Sci. USA, 75, 280-284, 1978). On the other hand, it is reported that oligodeoxynucleotide is rapidly decomposed in culture of mammal cells, cell extracts or sera (Wickstrom, E., Journal of Biochemical and Biophysical Methods, 13, 97 102, 1986). Accordingly, in order to expect a more effective antiviral activity of oligodeoxynucleotide, it is desired that the oligodeoxynucleotide be converted into such derivatives that are not decomposed. It is reported that for example, derivatives obtained by converting the phosphodiester in oligodeoxynucleotide into methylphophonate inhibit proliferation of herpes simplex virus type I (Smith, C.C., et al., Proc. Natl. Acad. Sci. USA, 83, 2787-2791, 1986). It is also reported that derivatives obtained by converting the phosphodiester in oligodeoxynucleotide into phosphorothioate have an improved stability (Stein, C.A., Nucleic Acids Res., 16, 3209-3221, 1988). It is further reported that these derivatives inhibit proliferation of AIDS virus (Matsukura, M., et al., Proc. Natl. Acad. Sci. USA, 84, 7706-7710, 1987). It is further reported that by leading to phosphorothioate derivatives in double stranded poly RNA or an interferon inducer, antiviral activity against bovine vesicular stomatitis virus is enhanced (De Clercq, E., et al., Virology, 42, 421-428, 1970).

On the other hand, it is known that 2′-O-methylribooligonucleotide takes a structure of the 2′-hydroxy group in the sugar moiety of RNA being methylated and has the property showing resistance to various enzymes (nucleases) for decomposing DNA or RNA (Gray, M.W., et al., Biochem. Biophys. Acta, 134, 243, 1967; Dunlap, B.E., et al., Biochemistry, 10, 2581-2587, 1971). Furthermore, 2′-O-methylribooligonucleotide has the property that it forms a stable duplex with RNA having its complementary sequence and the stability is significantly better than the stability of DNA-RNA complementary double strand having the same nucleotide sequence (Inoue, H., et al., Nucleic Acids Res., 15, 6131-6148, 1987). It is also known that 2′-O-methylribooligonucleotide is used for site-specific cleavage of RNA, utilizing the property resistant to decomposition from RNase H (Inoue, H., et al., FEBS Letters, 215, 327-330, 1987). In addition, 2′-O-methylribooligonucleotide is used to produce a unidirectional deletion mutant of DNA, utilizing the property showing significant resistance to DNase Ba131 nuclease as compared to DNA (Mukai, S., et al., Nucleic Acids Symposium Series, No. 19, 117-120, 1988). The foregoing findings are based on the characteristics that 2′-O-methylribooligonucleotide forms a stable duplex with RNA having its complementary nucleotide sequence and shows resistance various enzymes (nucleases) for decomposing DNA or RNA. Moreover, it was reported in the past that poly-2′-O-methylribooligonucleotide inhibited the progress of cancer in mice caused by retro virus, while its mechanism was clearly unknown (Tennant, R.W., et al., Proc. Natl. Acad. Sci., USA, 71, 3167-3171, 1974). In addition, 2′-O-methylribooligonucleotide can be prepared as in oligoribonucleotide using less expensive raw materials than in deoxyoligonucleotide.

With respect to the structure and properties of AIDS virus, the following findings have been made according to recent studies. That is, AIDS virus is retro virus having a single stranded RNA of about 9000 nucleotides in length as the substance of gene and has reverse transcriptase. Its nucleotide sequence (primary structure) of gene is already reported (Ratner, L., et al., Nature, 313, 277-284, 1985; Muesing, M.A., et al., Nature, 313, 450-458, 1985; Sanchez-Pescador, R., et al., Science, 227, 484-492, 1985; Wain-Hobson, S., et al., Cell, 40, 9-17, 1985). Target cells of infection are helper/inducer cells which are T4 (antigen detected with a monoclonal antibody) positive as surface antigen among human lymphocytes and after infection, cause these cytopathic effects and break down. The virus after adsorption to and invasion into the cells changes to linear double stranded viral DNA by reverse transcriptase possessed by themselves utilizing tRNA^{Lys} as a primer, transfers into the nucleus to take a circular structure and is integrated into host cell chromosomal DNA to become provirus. Transcription from the provirus is effected by cell-derived RNA polymerase II as in messenger RNA (hereafter simply referred to as "mRNA") of host cells, whereby the provirus is translated into viral protein. This procedure is regulated by at least two gene products, i.e., tat (transactivator) and rev (regulator of expression of virion proteins) encoded by the virus per se. tat works as a transcription activation factor for virus upon the region called TAR element (transacting responsive element) immediately after the initiation site of transcription (Rosen, C.A., et al., Cell, 41, 813-823, 1985). The transcribed viral RNA becomes in part genome RNA encapsulated into virion and is in part utilized as mRNA for expressing gene. Several species of mRNA are present; RNA in full length for expressing viral antigen and reverse transcriptase, mRNA for expressing envelope glycoprotein which is shortened by being spliced once, mRNA which is further shortened by being spliced at least twice in order to express genes such as tat, rev, etc., and the like.

As described above, the present invention has been successful for inhibiting infection with and proliferation of AIDS virus by further derivating the phosphate moiety, taking advantages of these 2′-O-methylribooligonucleotide and oligoribonucleotide. It has also been made clear that the inhibitory effect is exhibited in the binding mode of 3'-5' bond in the monomer of these oligomers, that is, 3'-O-methylnucleoside may be its constituent component. Accordingly, the 3'-5' bond in the sugar is contained in the monomer of the oligomer molecule. Further, the 2'-hydroxy group may be all or in part methylated or acylated. The sugar at the 3'- and 5'-termini in the oligonucleotide may optionally have an substituent as shown in general formulae (1) through (4).

As described above, the compound of the present invention is in the form that the phosphate in the oligomer is derivated. As a result of various investigations, it has been found that the derivatives containing a thiophosphate bond is particularly effective and the thiophosphate bond is not necessary for all of the phosphodiester bonds in the oligomer molecule. It has also been found that the inhibitory effect can be exhibited in any of the case that these nucleotide sequences are complementary to those of genomic RNA for AIDS virus or DNA integrated into chromosome and in the case that the nucleotide sequences are not necessary complementary.

In the present invention, the oligomer having a complementary sequence should preferably take as the target a region that plays an important role on viral gene. For example, the derivative (Compound VI) recited in claim 9, the derivative (Compound VII) recited in claim 10, the derivative (Compound VIII) recited in claim 11, the derivative (Compound IX) recited in claim 12 and the derivative (Compound XII) recited in claim 15 are all sequences complementary to the region having a nucleotide sequence: 5' U U U A U C C A U U U U C A G A A U U G G G U C U 3' which is present after 5300th (the initiation site of transcription is counted as the first) of viral genomic RNA or mRNA, and the region in which cleavage and ligation of the first splicing of mRNA occur. Therefore, by obstructing the same, it can be expected that expression of envelope protein or genes such as tat, rev, etc. is inhibited. Furthermore, the derivative (Compound X) recited in claim 13 is complementary to a nucleotide sequence: 5' C A G U G G C G C C C G A A C A G G G A C 3' including the primer-tRNA binding site present in the region for initiating reverse transcription of viral genomic RNA. Accordingly, hindrance of initiating transcription that is the first stage of replicating viral gene can be expected.

Further, the derivative (Compound XI) recited in claim 14 is complementary to a nucleotide sequence in TAR element upon which the tat product: 5' C A G A U C U G A G C C U G G G A G C U C 3' present around the 5′-terminal of viral genomic RNA or mRNA works;

The derivative (Compound XXVII) recited in claim 3 is complementary to a nucleotide sequence present downstream from the primer binding site on viral genomic RNA and, it can be expected to inhibit transcription and replication of virus.

The derivative (Compound XXVIII) recited in claim 4 is complementary to a nucleotide sequence right upstream gag gene initiation codon.

The derivative (Compound XXIX) recited in claim 5 is complementary to a nucleotide sequence in gag gene and, it can be expected to inhibit translation of gag protein.

It is noted that all these derivatives show a potent anti-AIDS viral activity, although mechanism on the activity has not been experimentally proven in detail.

On the other hand, as is described in publications (cf., Matsukura, M., et al., Proc. Natl. Acad. Sci. USA, 84, 7706-7710, 1987; Agrawal, S., et al., Proc. Natl. Acad. Sci. USA, 85, 7709-7083, 1988) regarding anti-AIDS viral activity using oligodeoxynucleotide derivatives, it is reported that oligodeoxynucleotide derivatives not complementary to the nucleotide sequence of AIDS viral genomic RNA or DNA integrated into chromosome also possess anti-AIDS viral activity, like the complementary derivatives.

On the other hand, the compounds represented by general formulae (1), (3) and (4) in the present invention which can be produced using less expensive raw materials than in oligodeoxynucleotide derivatives exhibit the anti-AIDS viral activity, although these compounds are not necessarily complementary to the nucleotide sequence of AIDS viral genomic RNA or DNA integrated into chromosome. It has been made clear that among the compounds represented by general formula (1), for example, the derivatives (Compound XXXII) recited in claim 6, exhibit the anti-AIDS viral activity, although these compounds are not complementary to the nucleotide sequence of AIDS viral genomic-RNA or DNA integrated into chromosome. It has also been made clear that for example, the derivatives (Compound XV) recited in claim 17, the derivatives (Compound XVI) recited in claim 18, the derivatives (Compound XVII) recited in claim 19 and the derivatives (Compound XVIII) recited in claim 20, among the compounds represented by general formula (3), and among the compounds represented by general formula (4), for example, the derivatives (Compound XXVI) recited in claim 33 exhibit the anti-AIDS viral activity, although these compounds are not complementary to the nucleotide sequence of AIDS viral genomic RNA or DNA integrated into chromosome.

On the other hand, the compounds in the present invention have low molecular weights unlike high molecular weight double stranded RNA known as a potent interferon inducer (cf., Lanpson, G.P., et al., Proc. Natl. Acad. Sci. USA., 58, 782, 1967; Field, A.K., et al., Proc. Natl. Acad. Sci. USA., 58, 1004, 1967) and derivatives thereof (De Clercq, E., et al., Virology, 42, 421-428, 1970). The compounds in the present invention are also clearly distinguished over 2'-5' oligoA all composed of adenosines and containing 2'-5' bond which is a protein synthesis inhibitor found in cells treated with interferon (cf., Kerr, I.M., Proc. Natl. Acad. Sci. USA., 75, 256, 1978) and derivatives thereof.

The oligoribonucleotide derivatives of the present invention can be produced by known methods such as the amidite method (cf., Matteucci, M.D., et al. Tetrahedron Lett., 22, 719, 1980), the H-phosphonate method (cf., Froehler, B.C. et al. Tetrahedron Lett., 27, 469, 1986; Garegg, P.J., et al., Tetrahedron Lett., 27, 4055, 1986) and the like. The 2'-O-methylribonucleotide derivatives represented by general formula (5) which can be provided as raw materials for producing the 2'-O-methylribooligonucleotide derivatives according to the H-phosphonate method can be produced by leading to the protected 2'-O-methylribonucleoside derivatives by known methods (cf., Robins, M.J., et al., J. Org. Chem., 39, 1891, 1974; Heikkila, J., et al., Acta Chem. Scand., B36, 715, 1982; Inoue, H., et al., Nucleic Acids Research, 15, 6131, 1987) and then reacting with phosphorus trichloride by known method (cf., Froehler, B.C., et al., Tetrahedron Letters, 27, 469-472, 1986; Garegg, P.J., et al., Tetrahedron Letters, 27, 4051-4054, 1986). According to the process, Compounds I, II, III, IV and V shown below were produced. 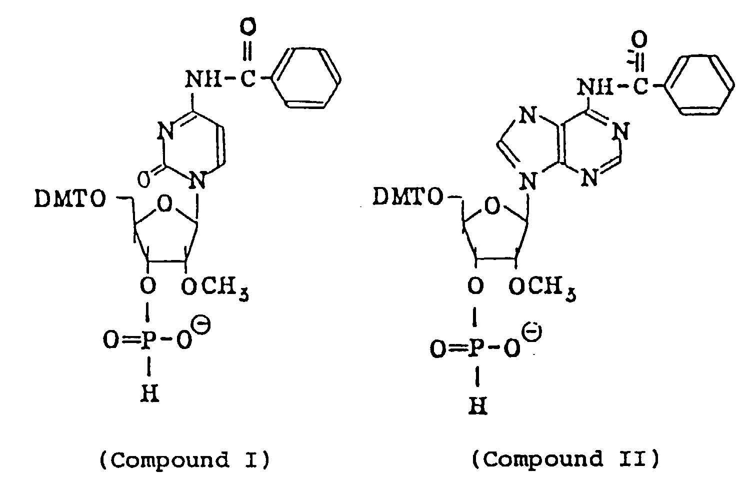 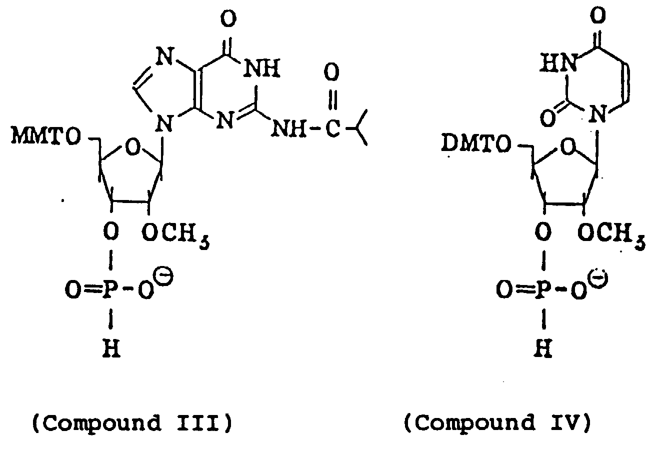 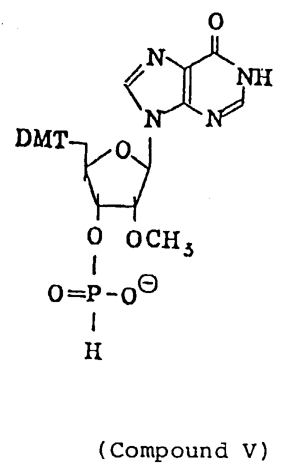 wherein DMT and MMT represent dimethoxytrityl group and monomethoxytrityl group, respectively.

Further the 2′-O-methylribonucleotide derivatives represented by general formula (5) can also be produced by reacting the protected 2′-O-methylribonucleoside derivatives with phosphorous acid in the presence of a condensation reagent such as triisopropylbenzenesulfonyl chloride or pivaloyl chloride by known methods (cf., Hata, T., et al., J. Am. Chem. Soc., 96, 7363, 1974; Sekine, M., et al., Tetrahedron Letters, 29, 1037-1040, 1988). Furthermore, the protected 3′-O-methylnucleoside derivatives are produced according to known methods (cf., Robins, M.J., et al., J. Org. Chem., 39, 1891, 1974; Heikkila, J., et al., Acta Chem. Scand., B36, 715, 1982) and the derivatives can be led to the 3′-O-methylnucleoside-H-phosphonate derivatives by the process described above, which can be provided as raw materials for producing the oligoribonucleotide derivatives represented by general formulae (1) and (4).

In the oligoribonucleotide derivatives represented by general formulae (1) and (4) wherein all or any of the substituents shown by X₁ - Xₘ₊₁ or X₁′ - X′_{m‴+1} are hydrogen, raw materials for producing the derivatives are, for example, protected 2′(or 3′)-O-(tert-butyldimethylsi yl)ribonucleosides, which can easily be produced by a known method (Ogilvie, K.K., Can. J. Chem., 51, 3799, 1973); these derivatives can be led to the H-phosphonate derivatives in a manner similar to described above.

The novel nucleoside derivatives represented by general formula (6) can be produced, for example, by a known method (cf., Miyoshi, K., et al., Nucleic Acids Res., 8, 5491, 1980) using the protected 3′-O-methyl (3′-O-alkyl or 3′-deoxy)nucleosides.

Using the protected novel 2′-O-methylribonucleotide derivatives, protected 3′-O-methylribonucleotide derivatives, protected 2′(or 3′)-O-(tert-butyldimethylsilyl)ribonucleotide derivatives and novel nucleoside derivatives produced by the foregoing processes, the 2′-O-methylribooligonucleotide derivatives and oligoribonucleotide derivatives can be produced, for example, by known methods (cf., Froehler, B.C., et al., Tetrahedron Letters, 27, 469-472, 1986; Garegg, P.J., et al., Tetrahedron Letters, 27, 4051-4054, 1986). That is, the nucleotide derivative elements are sequentially condensed novel nucleoside derivatives as the polymer support, having acyl chloride to elongate the chain. Then, oxidation is performed using various oxidizing agents such as sulfur (S₈), iodine (I₂) or an alkylamine/carbon tetrachloride, etc. followed by removal of the protective group and purification.

Further in the compounds represented by general formulae (1), (2) and (4) wherein the substituents shown by Q, Q′ and Q‴ are thio anions or thio anions and oxo anions and in the compounds represented by general formula (3), these compounds can also be produced by the amidite method (cf., Matteucci, M.D., et al., Tetrahedron Lett., 22, 719, 1980; Shibahara, S., et al., Nucleic Acids Res., 15, 4403, 1987; Usman, N., et al., J. Am. Chem. Soc., 109, 7845, 1987). In this case, the monomer may be oxidized with (S₈) or iodine (I₂) in the oxidation step after the condensation and then subjected to chain elongation followed by removal of the protective group and purification.

Further in the compounds which contain at the 5′-terminal thereof, for example, an alkyl-substituted thiophosphoryl group, an alkyl-substituted phosphoryl group or an alkyl-substituted alkylaminophosphoryl group, the chain elongation of the oligomer is effected on the polymer support by the process described above; after completion of the oxidation, the oxidation product is condensed with an alkyl-H-phosphonate and the condensation product is oxidized with sulfur (S₈), iodine (I₂) or an alkylamine/carbon tetrachloride followed by removal of the protective group and purification. The derivatives in which cholesterol is bound at the 5′-terminal via a spacer can be likewise obtained by condensing with mono(monomethoxytritylated) alkanediol H-phosphonate, treating with an acid, condensing with cholesteryl-H-phosphonate, oxidizing, removing the protective group and purification.

On the other hand, the compounds containing, for example, a hydroxyalkylphosphoryloxy group or a hydroxyalkylthiophosphoryloxy group at the 3′-terminal thereof can be obtained by condensing, oxidizing, removing the protective group and purification in a similar manner, using as a starting material a polymer support having bound thereto an alkanediol monosuccinate.

The anti-AIDS viral activity can be determined by the method discribed in a publication (cf., Harada, S., et al., Science, 229, 563-566, 1985), using HTLV-III_{β} (cf., Popovic, M., et al., Science, 224, 497-500, 1984) which is an isolated strain of AIDS virus. According to this method, MT-4 cells or HTLV-I positive cell line is used as the target cell so that this method is highly sensitive to viral infection and can exhibit the cytopathic effect highly efficiently. Using this method, the cytopathogen inhibitory effect was examined with Compounds VI, VII, VIII, IX, X, XI, XII, XV, XVI, XVII, XVIII, XXIII and XXIV, after each compound was diluted with medium into various concentrations. As the result, the cytopathogen inhibitory effect and the effect of inhibiting development of viral specific antigen positive cells were noted with Compound VI in a concentration of 10 µM, Compounds VII, X and XII in a concentration of 15 µM, and Compound VIII in a concentration of 1 µM. No cytotoxicity was detected even in the maximum concentration of 120 µM tested. On the other hand, the cytopathogen inhibitory effect and the effect of inhibiting development of viral specific antigen positive cells were noted with Compound IX in a concentration of 240 µM and Compound XI in a concentration of 30 µM. No cytotoxicity was detected in these concentrations.

Similarly, these inhibitory effects were noted with Compound XVI in a concentration of 1 µM. On the other hand, these inhibitory effects were noted with Compound XV in a concentration of 7.5 µM, Compound XVII in a concentration of 15 µM and Compound XVIII in a concentration of 120 µM. No cytotoxicity was detected with these compounds even in the maximum concentrations (60 µM with Compounds XV and XVI and 120 µM with Compounds XVII and XVIII).

Further Compound XXIII substantially completely inhibited cytopathogen in a concentration of 3.8 µM and appearance of viral specific antigen positive cells in a concentration of 7.5 µM, respectively. Compound XXIV substantially completely inhibited cytopathogen in a concentration of 7.5 µM and appearance of viral specific antigen positive cells in a concentration of 30 µM, respectively. No cytotoxicity was detected with these compounds even in the maximum concentrations (60 µM).

On the other hand, no cytopathogen inhibitory effect was recognized with deoxyoligocytidine phosphorothioate (15 mer) or deoxyoligonucleotide (phosphate type) having the same nucleotide sequence as in Compound VI or XI in a concentration of 10 µM.

As is clear from the foregoing, the novel 2′-O-methylribooligonucleotide derivatives and novel oligoribonucleotide derivatives in accordance with the present invention have the anti-AIDS viral activity and are expected to use as anti-AIDS drugs.

The antiviral agent of the present invention may be used in a range of 0.01 to 2,000 mg, preferably 0.02 to 500 mg.

The effective component may be used in the form of, for example, a capsule, an elixir, a microcapsule or a suspension.

The compound used as the effective component of the present invention can be administered to the patient who requires treatment or prophylaxis, in a dose of 0.01 to 1,000 mg per person, generally several times in a daily dose of 0.02 to 2,000 mg. The dose can be varied depending upon condition of disease, body weight of the patient and other factors recognized by one skilled in the art.

The compound used in the present invention or physiologically acceptable salt compound or a mixture thereof can be mixed in an amount of approximately 0.2 to 500 mg together with vehicles, carriers, diluents, binders, antiseptics, stabilizers, flavors, etc. in a unit dose form required to make pharmaceutical preparations generally admitted. An amount of the active substance in these compositions or preparations is incorporated to give an appropriate dose in a range indicated.

Specific examples of drugs which can be incorporated into tablets, capsules, etc. are given below: binders such as tragacanth, gum arabic, corn starch or gelatin; excipients such as fine crystalline cellulose; swelling agents such as corn starch, pregelatinized starch, alginic acid, etc.; lubricants such as magnesium stearate; sweeteners such as sucrose, lactose or saccharine; flavors such as peppermint, Gaultheria adenothrix oil or cherry, etc. In case that the preparation unit form is a capsule, a liquid carrier such as oils and fats can additionally be incorporated into the materials of type described above. A variety of other materials may be incorporated as protectives or in order to change physical form of the preparation unit by another method. For example, tablets can be coated with shellac or sugar or with both of them. Syrup or elixir may contain sucrose as a sweetener, methyl- and propylparabens as antiseptics and pigments and flavors such as cherry or orange flavor.

In the case of enteric coating, for example, 8% aqueous solution of hydroxyphenylmethyl cellulose is used as a coating. pretreatment agent, 10% aqueous solution of hydroxypropylmethyl cellulose phthalate and 3% aqueous solution of polyacetyne are used as coating agents. They are used respectively to make enteric coating preparations in a conventional manner.

A sterile composition for injection can be formulated in a conventional manner to prepare pharmaceutical preparations by dissolving or suspending the active substance, naturally occurring plant oils such as sesame oil, coconut oil, peanut oil, cotton seed oil, etc. or synthetic fatty vehicle such as ethyl oleate in a vehicle such as water for injection. Buffer agents, antiseptics, antioxidants, etc. may be added, if necessary.

### Examples

Hereafter the present invention is concretely described by referring to the examples below.

### Example 1 Production of N⁴-benzoyl-5′-O-dimethoxytrityl-2′-O-methylcytidine-3′-O-(H-phosphonate) (Compound I):

To 50 ml of a solution of 436 µl (5 mmols) of phosphorus trichloride and 5.5 ml (50 mmols) of N-methylmorpholine in dichloromethane was added 1.197 g (17.3 mmols) of 1,2,4-triazole. The mixture was stirred at room temperature for 30 minutes. To the mixture was added 17 ml of a solution of 664.7 mg (1 mmol) of N⁴-benzoyl-5′-O-dimethoxytrityl-2′-O-methylcytidine in dichloromethane over 10 minutes. The mixture was further stirred at room temperature, whereby the progress of the reaction was monitored by thin layer chromatography (mobile phase: dichloromethane/2% triethylamine/8% methanol) using silica gel 60. After it was confirmed that the raw material spot (Rf value: 0.60) disappeared and a new spot derived from Compound I appeared at Rf value of 0.41, 40 ml of triethylamine-bicarbonate buffer (1 M, pH 7.5) was added. The aqueous layer was extracted with 15 ml of dichloromethane. The dichloromethane layers were combined, dried over anhydrous sodium sulfate and then evaporated to dryness. Next, the resulting foamy residue was dissolved in 3 ml of dichloromethane containing 2% triethylamine and the solution was. purified by passing through a column packed with 24 g of silica gel 60. That is, elution was carried out using 100 ml of dichloromethane/2% triethylamine, then 200 ml of dichloromethane/2% triethylamine/3% methanol and further 200 ml of dichloromethane/2% triethylamine as the mobile phase and the pure fraction of Compound I obtained was evaporated to dryness. The resulting residue was dissolved in 40 ml of dichloromethane and the solution was washed with 15 ml of 1 M triethylamine-bicarbonate buffer (pH 7.5). After drying over anhydrous sodium sulfate, the dichloromethane layer was evaporated to dryness to give 703 mg (0.849 mmol) of Compound I in yield of 84.9%.

In a manner similar to the operation described above, N⁶-benzoyl-5′-O-dimethoxytrityl-2′-O-methyladenosine-3′-O-(H-phosphonate) (Compound II), N²-isobutylyl-5′-O-monomethoxytrityl-2′-O-methylguanosine-3′-O-(H-phosphonate) (Compound III), 5′-O-dimethoxytrityl-2′-O-methyluridine-3′-O-(H-phosphonate) (Compound IV), 5′-O-dimethoxytrityl-2′-O-methylinosine-3′-O-(H-phosphonate) (Compound V) were obtained in yields of 93.9%, 49.4%, 77.4% and 56.0%, respectively.

Next, ³¹P-NMR of these compounds was measured. With respect to Compounds I, II and III, a part of each compound was dissolved in dichloromethane and after mixing the solution with an equimolar amount of 1,8-diazabicyclo[5.4.0]undec-7-ene (simply referred to as DBU), the mixture was dropped into n-hexane to form the DBU salt, the salt was ground into powders and the powders were measured. On the other hand, Compounds IV and V were measured as they were, i.e., as the triethylamine salts. As a solvent for the measurement, d₅ pyridine was used. D₂O solution of phosphoric acid was used as the external standard. Their chemical shifts (ppm) were as follows.

| | |
|---|---|
| Compound I | -1.79 ppm |
| Compound II | -2.20 ppm |
| Compound III | -2.04 ppm |
| Compound IV | -0.73 ppm |
| Compound V | -0.40 ppm |

### Example 2 Production of 5'-O-dimethoxytrityl-2'-O-(tert-butyldimethylsilyl)inosine-3'-O-(H-phosphonate) (Compound XXI) and 5'-O-dimethoxytrityl-3'-O-(tert-butyldimethylsilyl)inosine-2'-O-(H-phosphonate) (Compound XXII)

5'-O-Dimethoxytritylinosine, 28.5 g (50 mmols), synthesized in a conventional manner was dissolved in 100 ml of pyridine and 9.5 g (140 mmols) of imidazole and 9.9 g (66 mmols) of tert-butyldimethylchlorosilane were added to the solution. The mixture was stirred at room temperature. Five hours after, the progress of the reaction was monitored by thin layer chromatography (mobile phase: chloroform/methanol **=** 10 : 1) using silica gel 60. After it was confirmed that the raw material spot (Rf value: 0.08) disappeared and new spots of 5'-O-dimethoxytrityl-2'-O-(tert-butyldimethylsilyl)inosine (Compound XIX) and 5'-O-dimethoxytrityl-3'-O-(tert-butyldimethylsilyl)inosine (Compound XX) newly appeared at Rf values of 0.44 and 0.33, respectively, 150 ml of water was added under chilling with ice water. The mixture was extracted once with 350 ml and twice with 100 ml of dichloromethane. The dichloromethane layers were combined, washed twice with 150 ml water and then concentrated. After the resulting oily residue was co-evaporated together with toluene, it was dissolved in 50 ml of dichloromethane and the solution was purified by passing through a column packed with 350 g of silica gel 60. That is, elution was performed using 7 liters of dichloromethane in which an acetone concentration was stepwise increased from 2% (v/v) to 13% (v/v) as the mobile phase. Pure fractions of Compound XIX eluted in acetone concentrations of 2% to 5% were collected and evaporated to dryness (4.05 g, 5.89 mmols). Next, the mixture of Compound XIX and Compound XX eluted in acetone concentrations of 6 to 13% was evaporated to dryness. The residue was again purified by passing through a column packed with 350 g of silica gel 60. That is, elution was performed using as the mobile phase 6 liters of dichloromethane in which an acetone concentration was stepwise increased from 1% (v/v) to 12% (v/v) and then in acetone concentrations of 7% to 12% using 2.5 liters of ethyl acetate to give the pure fraction of Compound XIX. The fractions of a mixture of Compound XIX and Compound XX eluted with ethyl acetate and the pure fraction of Compound XX were evaporated to dryness and dried under reduced pressure, respectively. Yielded amount of Compound XIX was 4.3 g, 6.26 mmols; when combined with the isolate of first column chromatography, 8.35 g, 12.15 mmols, showing yield of 24.3%. The mixture of Compound XIX and Compound XX was obtained in an amount of 1.13 g, 1.65 mmols in yield of 3.3% and, Compound XX was obtained in an amount of 2.66 g, 3.88 mmols, in yield of 7.8%.
Compound XIX: FAB mass spectrum, 685 (M-H⁺);
¹H-NMR spectrum (CDCl₃, δ ppm)
8.03 (s, H₈ or H₂), 7.99 (s, 1, H₂ or H₈), 7.45-6.81 (m, 13, Ar-H), 6.01 (d, 1, H₁′), 4.89 (m, 1, H₂′), 4.33 (m, 1, H₃′), 4.27 (m, 1, H₄′), 3.79 (s, 6, MeO), 3.45 (m, 2, H₅′), 2.71 (d, 1, OH₃′), 0.85 (s, 9, tert-Bu-Si), 0.10 (s, 3, Me-Si), -0.125 (s, 3, Me-Si);
UV absorption spectrum (ethanol) λmax = 247nm, λmin = 223nm, λ270 nm-shoulder.
Compound XX : FAB mass spectrum, 685 (M-H⁺);
¹H-NMR spectrum (CDCl₃, δ ppm)
8.11 (s, H₈ or H₂), 8.05 (s, 1, H₂ or H₈), 7.44-6.80 (m, 13, Ar-H), 5.98 (d, 1, H₁′), 4.62 (m, 1, H₂′), 4.50 (m, 1, H₃′), 4.18 (m, 1, H₄′), 3.77 (s, 6, MeO), 3.38 (m, 2, H₅′), 3.07 (d, 1, OH₂′), 0.89 (s, 9, tert-Bu-Si), 0.09 (s, 3, Me-Si), 0.02 (s, 3, Me-Si);
UV absorption spectrum (ethanol) λmax = 247nm, λmin = 223nm, λ270 nm-shoulder.

Proton of the sugar moiety in the ¹H-NMR spectrum was identified by the spin decoupling method.

### Device for measurement:

FAB mass spectrum: JEOL JMS-DX300 (manufactured by JEOL, Ltd )
¹H-NMR spectrum: Varian XL-300, 300 MHz
UV absorption spectrum: Hitachi 320 Spectrophotometer

Next, 0.872 ml (10 mmols) of phosphorus trichloride was added to a solution of 11 ml (100 mmols) of N-methylmorpholine and 2.38 g (34.5 mmols) of 1,2,4-triazole in dichloromethane. The mixture was stirred at room temperature for 30 minutes. To the liquid mixture was added 32 ml of a solution of 1.37 g (2 mmols) of 5′-O-dimethoxytrityl-2′-O-(tert-butyldimethylsilyl)-inosine (Compound XIX) in dichloromethane-dimethylformamide (15 : 1, v/v) over 10 minutes. After stirring at room temperature for further 20 minutes, the progress of the reaction was monitored by thin layer chromatography (mobile phase: dichloromethane/2% triethylamine/8% methanol) using silica gel 60. After it was confirmed that the raw material spot (Rf value: 0.64) disappeared and a spot derived from 5′-O-dimethoxytrityl-2′-O-(tert-butyldimethylsilyl)inosine-3′-O-(H-phosphonate) (Compound XXI) appeared at Rf value of 0.44, 80 ml of 1M triethylamine-bicarbonate buffer (pH 7.5) was added. The aqueous layer was extracted with 40 ml of dichloromethane. The dichloromethane layers were combined, dried over anhydrous sodium sulfate and then evaporated to dryness. The procedure up to this stage was repeated once again on the same scale. The resulting foamy residues obtained by both procedures were combined and dissolved in 10 ml of dichloromethane containing 2% triethylamine. The solution was purified by passing through a column packed with 40 g of silica gel 60. That is, using as the mobile phase, in sequence, 150 ml of dichloromethane/2% triethylamine, 200 ml of dichloromethane/2% triethylamine/2% methanol, 200 ml of dichloromethane/2% triethylamine/4% methanol, 200 ml of dichloromethane/2% triethylamine/6% methanl, 200 ml of dichloromethane/2% triethylamine/8% methanol and 100 ml of dichloromethane/2% triethylamine/9% methanol, elution was carried out and the pure fraction of Compound XXI thus obtained was evaporated to dryness. The resulting residue was dissolved in 50 ml of dichloromethane and the solution was washed with 40 ml of 1 M triethylamine-bicarbonate buffer (pH 7.5). After drying over anhydrous sodium sulfate, the dichloromethane layer was evaporated to dryness to give 3.0 g (3.53 mmols) of Compound XXI in yield of 88%.

5′-O-Dimethoxytrityl-3′-O-(tert-butyldimethylsilyl)inosine-2′-O-(H-phosphonate) (Compound XXII) was isolated in a manner similar to above in yield of 72.5%.
Compound XXI: FAB mass spectrum, 749 (M-H⁺);
³²P-NMR spectrum (d₅-pyridine, δ ppm, external standard 5% H₃PO₄ in D₂O);
-1.469, ¹J_{P-H} = 608.7Hz, ³J_{P-H} = 10.2Hz;
¹H-NMR spectrum (CDCl₃, δ ppm)
8.40 (s, H₈ or H₂), 8.00 (s, 1, H₂ or H₈), 7.44-7.18 (m, 13, Ar-H), 7.07 (d, 1, P-H, J=625Hz), 6.32 (d, 1, H₁′), 4.82 (m, 1, H₂′), 4.85 (m, 1, H₃′), 4.46 (m, 1, H₄′), 3.78 (s, 6, MeO), 3.47 (m, 2, H₅′), 0.72 (s, 9, tert Bu-Si), 0.01 (s, 3, Me-Si), -0.21 (s, 3, Me-Si).
Compound XXII: FAB mass spectrum, 749 (M-H⁺);
³²P-NMR spectrum (d₅-pyridine, δ ppm, external standard 5% H₃PO₄ in D₂O):
-1.858, ¹J_{P-H} = 612.8HZ, ³J_{P-H} = 10.7Hz;
¹H-NMR spectrum (CDCl₃, δ ppm)
7.98 (s, H₈ or H₂), 7.93 (s, 1, H₂ or H₈), 7.41-6.77 (m, 13, Ar-H), 6.95 (d, 1, P-H, J=634Hz), 6.23 (d, 1, H₁'), 5.31 (m, 1, H₂'), 4.50 (m, 1, H₃'), 4.23 (m, 1, H₄'), 3.76 (s, 6, MeO), 3.31 (m, 2, H₅'), 0.86 (s, 9, tert-Bu-Si), 0.14 (s, 3, Me-Si), 0.03 (s, 3, Me-Si).

### Example 3 Production of 5'-O-monomethoxytrityl-N²-isobuty)yl-3'-O-methylguanosine-bound controlled pore glass:

5'-O-monomethoxytrityl-N²-isobutylyl-3'-O-methylguanosine synthesized in a conventional manner was led to 2'-O-succinylpentachlorophenyl ester by a known process (cf., Miyoshi, K., et al., Nucleic Acids Res., 8, 5491, 1980). That is, 639 mg (1 mmol) of 5'-O-monomethoxytrityl-N²-isobutylyl-3'-O-methylguanosine and 183 mg of 4-N,N-dimethylaminopyridine (merely referred to as DMAP) were dissolved in 4 ml of absolute pyridine. While stirring at room temperature, 150 mg (1.5 mmol) of succinic anhydride was added to the solution. After stirring for 90 minutes, the reaction liquid was distilled under reduced pressure. The resulting oily residue was dissolved in 35 ml of chloroform. The solution was washed 3 times with 30 ml of 0.1 M triethylamine-bicarbonate buffer (pH 7.5). After drying over anhydrous sodium sulfate, the chloroform layer was evaporated to dryness under reduced pressure. The obtained residue was dissolved in 5 ml of dimethylformamide (DMF) and 400 mg (1.5 mmol) of pentachlorophenol and 309 mg (1.5 mmol) of dicyclohexylcarbodiimide were added to the solution followed by stirring at room temperature for 13.5 hours. The precipitated insoluble matters were removed by filtration and the filtrate was removed by distillation under reduced pressure. The resulting residue was dissolved in 3 ml of chloroform and the solution was purified by column chromatography using a column packed with 20 g of silica gel 60. That is, elution was carried out using as the mobile phase, in sequence, 100 ml of chloroform, 100 ml of chloroform/0.3% methanol, 100 ml of chloroform/0.5% methanol and further 100 ml of chloroform/0.6% methanol and the resulting pure fractions of 5′-O-monomethoxytrityl-N²-isobutylyl 3′-O-methylguanosine-2′-O-succinyl-pentachlorophenyl ester were collected and evaporated to dryness under reduced pressure to give 530 mg of the ester in yield of 53.6% (Rf value: 0.73 when developed with chloroform : methanol = 20 : 1 in silica gel 60 thin layer chromatography). Next, the whole amount (530 mg) of the ester was dissolved in 5.4 ml of DMF; on the other hand, 1 g of controlled pore glass (CPG/long chain alkylamine, pore diameter: 500 Å, particle size: 122-177 µ, NH₂-: 30 µmols/g, manufactured by Pierce Chemical Inc.) was charged in a reactor equipped with a glass filter and washed with 1 ml of DMF followed by drying for a minute in an argon gas flow. The aforesaid pentachlorophenyl ester DMF solution, 5.4 ml, and 73 µl of triethylamine were added to the pore glass, respectively. After sealing, the reaction was carried out at 30°C overnight. The reaction liquid was filtered under an argon pressure. After washing 3 times with 5 ml of DMF and then 3 times with 5 ml of tetrahydrofuran (THF), the controlled pore glass was dried for a minute in an argon flow. Next, a liquid mixture of 410 mg of DMAP, 1.4 g of 2,6-lutidine, 660 µl of acetic anhydride and 6 ml of THF was added to the pore glass. The mixture was shaken at 30°C for 15 minutes to acetylate the unreacted amino group. After filtration, the reaction mixture was washed 3 times with 5 ml of THF and then 3 times with 5 ml of diethyl ether and dried in an argon flow to give 1 g of 5'-O-monomethoxytrityl-N²-isobutylyl-3'-O-methylguanosine-bound controlled pore glass. A small amount of the product was weighed and the monomethoxytrityl group was cleaved with 3% trichloroacetic acid. The isolated tritanol was subjected to quantitative colorimetry with a perchloric acid-ethanol mixture. As the result, the bound amount was 26 µmols/g.

Furthermore, 5'-O-dimethoxytrityl-N⁶-benzoyl-3'-O-methyladenosine-bound controlled pore glass, 5'-O-dimethoxytrityl-3'-O-methylinosine-bound controlled pore glass, 5'-O-dimethoxytrityl-3'-O-methyluridine-bound controlled pore glass, 5'-O-dimethoxytrityl-N4-benzoyl-3'-O-methylcytidine-bound controlled pore glass, 5'-O-dimethoxytrityl-2'-O-(tert-butyldimethylsilyl)inosine-bound controlled pore glass and monomethoxy tritylhexanediol-bound controlled pore glass were produced, respectively, in a manner similar to the above.

### Example 4 Production of 5′ Amp Cmp Amp Cmp Cmp Cmp Amp Amp Ump Ump Cmp Ump Gmp Amp Amp Amp Amp Ump Gmp Gm′ 3′ (Compound VI), wherein m represents 2′-O-methylnucleoside unit; m′ represents 3′-O-methylnucleoside unit; and P represents:

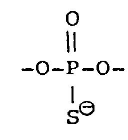

N²-Isobutylyl-5′-O-monomethoxytrityl-3′-O-methylguanosine-bound polymer support, 40 mg (binding amount: 1.0 µmol), was packed in a cartridge and the cartridge was set in a DNA synthesizer (model 380A, manufactured by Applied Biosystems Inc.). Following the procedure shown in Table 1, 2′-O-methylribonucleoside-3′-O-(H phosphonate) compounds (Compounds I through IV) were treated using III, IV, II, II, II, II, III, IV, I, IV, IV, II, II, I, I, I, II, I and II in this order. After repeating 19 cycles, the intermediate for preparing the polymer support was withdrawn from the cartridge and shifted to a reactor equipped with a glass filter. After washing with 1 ml of acetonitrile, the intermediate was dried in an argon gas flow. Next, 2 ml of a solution of 0.2 M sulfur (S₈) in triethylamine : carbon disulfide : pyridine (1 : 12 : 12, volume ratio) was added to the intermediate. After shaking, the mixture was allowed to stand for 12 hours. The reaction mixture was filtered and the intermediate for synthesizing the polymer support was washed 5 times with 2 ml of carbon disulfide and once with 2 ml of diethyl ether. After drying in an argon flow, the intermediate was transferred to a glass-made bial of a 3 ml volume. 28% Ammonia water, 2 ml, was charged in the bial and sealed followed by heating at 55°C for 5 hours. The polymer support was removed by filtration and the reaction liquid was evaporated to dryness under reduced pressure. The resulting residue was dissolved in 300 µl of 0.1 M triethyl ammonium-acetic acid buffer (pH 7.0, hereafter simply referred to as TEAA) containing 10% acetonitrile. The solution was purified through a column having a diameter of 1 cm and a length of 12 cm, packed with reverse phase silica gel (Waters Co., Ltd., Prep PAK-500/C-18). That is, 0.1 M TEAA buffer (pH 7.0) having a linear gradient of 10% to 35% acetonitrile was used as the mobile phase and quantitative determination was performed at absorbancy at a wavelength of 254 nm to give the fraction of Compound VI having a dimethoxytrityl group at the 5′-terminal. After the solvent was distilled off under reduced pressure, the residue was co-evaporated together with 2 ml of water 3 times and 3 ml of 80% acetic acid was added thereto followed by stirring for 10 minutes. After the reaction liquid was evaporated to dryness, the residue was further co-evaporated together with water to remove acetic acid. To the residue was added 1.5 ml of 0.1 M triethylamine-bicarbonate buffer (pH 7.5) containing 10% acetonitrile. The mixture was washed twice with 1.5 ml of diethyl ether. The aqueous layer was evaporated to dryness and 200 µl of 0.1 M TEAA buffer (pH 7.0) containing 10% acetonitrile was added to the resulting residue. After passing through a 0.45 µ filter (manufactured by Millipore Corp.), purification was performed by high performance liquid chromatography. That is, YMC Pack ODS (manufactured by Yamamura Science Co., Ltd.) column was used and 0.1 M TEAA buffer (pH 7.0) having a linear gradient of 10% to 70% acetonitrile was used as the mobile phase. The main peak eluted at a flow rate of 1.2 ml/min was fractionated to give 52.9 OD^{260 nm} units (about 226 nmols) of Compound VI in yield of 22.6%.

Compound VI was dissolved in D₂O containing 10 mM triethylamine-bicarbonate buffer (pH 7.5) and ³¹P-NMR was measaured (device for measurement: JEOL JMS-DX300, manufactured by JEOL Inc.). When trimethyl phosphate was used as the external standarad, Compound VI showed a characteristic signal in multiplet phosphorothioate at 51-55 ppm.

**Table 1**

| One Cycle of Procedure for Chain Elongation Reaction | | | |
|---|---|---|---|
| Operation Number | Name of Reagent or Operation | Liquid Feeding Time (second) | Number of Time |
| 1. | Washing with acetonitrile | 30 | 1 |
| 2. | Drying in argon | 20 | 1 |
| 3. | 3% Trichloroacetic acid/dichloromethane | 30 | 6 |
| 4. | Drying in argon | 5 | 1 |
| 5. | Washing with acetonitrile | 30+60 | 2 |
| | Drying in argon | 5+20 | |
| 6. | 0.2 M Compounds I-V, XV-XIII, XXI or XXII/acetonitrile 1.0 M Pivaloyl chloride/pyridine | 13 | 1 |
| 7. | Allowing to stand | 180 | 1 |
| 8. | Drying in argon | 15 | 1 |
| 9. | Washing with acetonitrile | 30 | 3 |
| | Drying in argon | 20 | |

Furthermore using 2 µmols of nucleoside-bound support as the starting material in a manner similar to the procedure described above, Compound VII was obtained in 26 OD^{260 nm} units (about 132 nmols) in yield of about 6.3% using, in sequence, III, IV, II, II, II, II, III, IV, I, VI, IV, II, II, I, I and I in the condensation cycle; Compound VIII was obtained in 41.5 OD^{260 nm} units (about 136 nmols) in yield of about 6.2%, using, in sequence, II, II, IV, II, III, III, IV, II, II, II, II, III, IV, I, IV, IV, II, II, I, I, I, II, I and II in the condensation cycle; Compound IX was obtained in 62 OD^{260 nm} units (about 528 nmols) in yield of about 24.2%, using, in sequence, II, III, IV, I, IV, IV, II, II and I in the condensation cycle; and Compound X was obtained in 37 OD^{260 nm} units (about 180 nmols) in yield of about 8.7%, using, in sequence, IV, I, II, I, I, III, I, III, III, III, I, IV, IV, III, IV, I, I, I, IV and III in the condensation cycle. Furthermore, using 1 µmol of nucleoside-bound support as the starting material, Compound XI was obtained in 16.0 OD^{260 nm} units (about 72 nmols) in yield of about 7.2%, using, in sequence, IV, I, IV, II, III, II, I, IV, I, III, III, II, I, I, I, IV, I, III, II and III in the condensation cycle.

Furthermore, in a manner similar to the procedure described above, Compound XV was obtained in 25 OD^{260 nm} units (about 81.7 nmols) showing yield of about 3.4%, using as the starting material 2.4 µmols of 5′-O-dimethoxytrityl-N⁶-benzoyl-3′-O-methyladenosine-bound controlled pore glass and using Compound II by 19 cycles in the condensation cycle; Compound XVI was obtained in 74 OD^{260 nm} units (about 301 nmols) showing yield of about 8.3%, using as the starting material 3.6 µmols of 5′-O-dimethoxytrityl 3′-O-methylinosine-bound polymer support and using Compound V by 19 cycles in the condensation cycle; Compound XVII was obtained in 58 OD^{260 nm} units (about 292 nmols) showing yield of about 8.1%, using as the starting material 3.6 µmols of 5′-O-dimethoxytrityl-3′-O-methyluridine-bound polymer support and using Compound IV by 19 cycles in the condensation cycle; and Compound XVIII was obtained in 25 OD^{260 nm} units (about 168 nmols) showing yield of about 4.7%, using as the starting material 3.6 µmols of 5′-O-dimethoxytrityl-N⁴-benzoyl-3′-O-methylcytidine-bound polymer support and using Compound I by 19 cycles in the condensation cycle; respectively.

In a similar manner, Compounds XXVII, XXVIII and XXIX were produced.

### Example 5 Production of Compound XII

In a reactor equipped with a glass filter was charged 120 mg (total binding amount, 3.12 µmols) of polymer support having bound thereto N²-isobutylyl-5′-O-monomethoxytrityl 3′-O-methylguanosine. Following the procedure shown in Table 1, 2′-O-methylribonucleoside 3′-O-(H-phosphonate) compounds Compounds I to IV) were used in the order of III, IV, II, II and II, which was repeated by 5 cycles. However, 2 ml each was used in operation Nos. 1, 3, 5, 6 and 9. Next, 2 ml of a solution of 0.2 M sulfur (S₈) in triethylamine : carbon disulfide : pyridine (1 : 12 : 12, volume ratio) was added to the system. After shaking, the mixture was allowed to stand for 70 minutes. The reaction mixture was filtered and the intermediate for synthesizing the polymer support was washed 5 times with 2 ml of carbon disulfide and 5 times with 2 ml of acaetonitrile followed by drying in an argon flow. Furthermore, the procedure was repeated by 9 cycles as shown in Table 1, using Compounds II, III, IV, I, IV, IV, II, II and I, in this order. Thereafter, 2 ml of a solution of 0.1 M iodine (I₂) in pyridine : N-methylimidazole : H₂O : THF (5 : 1 : 5 : 90, volume ratio) was added to the system. The mixture was allowed to stand at room temperature for 20 minutes. The reaction liquid was filtered and 2 ml of a solution of 0.1 M iodine (I₂) in triethylamine : H₂O : THF (5 : 5 : 90, volume ratio) was then added to the system. The allowing to stand at room temperature for 20 minutes, the reaction liquid was filtered. Furthermore, the procedure was repeated by 5 cycles as shown in Table 1, using Compounds I, I, II, I and II, in this order followed by the same sulfur oxidation treatment as described above overnight.

The reaction liquid was filtered and the intermediate for synthesizing polymer support was washed 5 times with 2 ml of carbon disulfide and once with 2 ml of diethyl ether. After drying in an argon flow, the intermediate was transferred to a glass-made bial of a 3 ml volume. 28% Ammonia water, 2 ml, was charged in the bial and sealed followed by heating at 55°C for 5 hours. The polymer support was removed by filtration and the reaction liquid was evaporated to dryness under reduced pressure. The resulting residue was dissolved in 300 µl of 0.1 M triethyl ammonium-acetic acid buffer (pH 7.0, hereafter simply referred to as TEAA) containing 10% acetonitrile. The solution was purified through a column having a diameter of 1 cm and a length of 12 cm, packed with reverse phase silica gel (Waters Co., Ltd., Prep PAK-500/C-18). That is, 0.1 M TEAA buffer (pH 7.0) having a linear gradient of 10% to 35% acetonitrile was used as the mobile phase and quantitative determination was performed at absorbancy at a wavelength of 254 nm to give the fraction of Compound XII having a dimethoxytrityl group at the 5′-terminal. After the solvent was distilled off under reduced pressure, the residue was co-evaporated together with 2 ml of water 3 times and 3 ml of 80% acetic acid was added thereto followed by stirring them for 10 minutes. After the reaction liquid was evaporated to dryness, the residue was further co-evaporated together with water to remove acetic acid. To the residue was added 1.5 ml of 0.1 M triethylamine-bicarbonate buffer (pH 7.5) containing 10% acetonitrile. The mixture was washed twice with 1.5 ml of diethyl ether. The aqueous layer was evaporated to dryness and 200 µl of 0.1 M TEAA buffer (pH 7.0) containing 10% acetonitrile was added to the resulting residue. After passing through a 0.45 µ filter (manufactured by Millipore Corp.), purification was performed by high performance liquid chromatography. That is, YMC Pack ODS (manufactured by Yamamura Science Co., Ltd.) column was used and 0.1 M TEAA buffer (pH 7.0) having a linear gradient of 10% to 50% acetonitrile was used as the mobile phase. The main peak eluted at a flow rate of 1.2 ml/min was fractionated to give 65.2 OD^{260 nm} units (about 278 nmols) of Compound XII in yield of 8.9%.

Compound XII was dissolved in D₂O containing 10 mM triethylamine-bicarbonate buffer (pH 7.5) and ³¹P-NMR was measaured (device for measurement: JEOL JMS-DX300, manufactured by JEOL Inc.). When trimethyl phosphate was used as the external standarad, Compound XII gave a characteristic signal in multiplet phosphorothioate at 51-55 ppm and a singlet signal derived from phosphate at -4.2 ppm in an integration ratio of 7 : 12. From the results of this NMR and the order for the oxidation at the chain elongation step, it has been made clear that Compound XII contains 5 phosphorus atoms from the 5′-terminal and 5 phosphorus atoms from the 3′-terminal, among which 2 phosphorus atoms in average are thiophosphates and the balance has phosphate diester bonds.

### Example 6 Production of Compound XXIII

5′-O-Dimethoxytrityl-2′-O-(tert-butyldimethylsilyl)inosine-CPG (controlled pore glass), 40 mg (binding amount: 1.0 µmol) each, was packed in 3 cartridges and the cartridges were set in a DNA synthesizer (model 380A, manufactured by Applied Biosystems Inc.). Following the procedure shown in Table 1, 5′-O-dimethoxytrityl-2′-O-(tert-butyldimethylsilyl)inosine-3′-O-(H-phosphonate) (Compound XXI) was used together with pivaloyl chloride. After repeating 19 cycles, the intermediate for preparing the polymer support was withdrawn from each cartridge and shifted to a reactor equipped with a glass filter. After washing with 1 ml of acetonitrile, the intermediate was dried in an argon gas flow. Next, 2 ml of a solution of 0.2 M sulfur (S₈) in triethylamine : carbon disulfide : pyridine (1 : 12 : 12, volume ratio) was added to the intermediate. After shaking, the mixture was allowed to stand for 2 hours. The reaction liquid was filtered and the intermediate for synthesizing the polymer support was washed 5 times with 2 ml of carbon disulfide and once with 2 ml of diethyl ether. After drying in an argon flow, the intermediate was transferred to a glass-made bial of a 3 ml volume. 28% Ammonia water, 1.5 ml, and 0.5 ml of ethanol were charged in the bial and sealed followed by heating at 55°C for 5 hours. The polymer support was removed by filtration and washed with 1 ml of ethanol and then with 1 ml of 50% ethanolic water. The filtrates were combined and evaporated to dryness. To the resulting residue was added 1.5 ml of THF solution (1 M) of tetra-(n-butyl)ammonium fluoride. The mixture was shaken at room temperature for 17 hours. Next, 1 ml of 20 mM triethyl ammonium-bicarbonate buffer (pH 7.0, hereafter simply referred to as TEAB) to dilute. The dilution was purified by gel filtration chromatography using a Sephadex G25-packed column (diameter of 1.6 cm, length of 5 cm, NAP-25, manufactured by Pharmacia Inc.). That is, after the reaction dilution described above was added, elution was performed using 20 mM TEAB buffer as the mobile phase and passed-through fractions were collected and evaporated to dryness. The residue was dissolved in 300 µl of 0.1 M triethylamine-acetate buffer (pH 7.0, hereafter simply referred to as TEAA) containing 10% acetonitrile and the solution was purified through a column packed with reverse phase silica gel Waters Co., Ltd., Prep PAK-500/C-18). That is, 0.1 M TEAA buffer having a linear gradient of 10% to 40% acetonitrile was used as the mobile phase and quantitative determination was performed at absorbancy at a wavelength of 254 nm to give the fraction of Compound XXIII having a dimethoxytrityl group at the 5′-terminal. After the solvent was distilled off, the residue was co-evaporated together with water 3 times and 3 ml of 80% acetic acid was added thereto followed by stirring for 10 minutes. After the reaction liquid was evaporated to dryness, the residue was further co-evaporated under reduced pressure together with water to remove acetic acid. To the residue was added 1.5 ml of 20 mM TEAB buffer. The mixture was washed twice with 1.5 ml of ethyl acetate. The aqueous layer was evaporated to dryness and 200 µl of 0.1 M TEAA buffer containing 10% acetonitrile was added to the resulting residue. The mixture was purified by high performance liquid chromatography. That is, YMC Pack ODS (manufactured by Yamamura Science Co., Ltd.; diameter of 6 mm, length of 30 cm) column was used and 0.1 M TEAA having a linear gradient of 10% to 50% acetonitrile was used as the mobile phase. The main peak eluted at a flow rate of 1.2 ml/min was fractionated to give 126 OD^{260 nm} units (about 514) nmols) of Compound XXIII in yield of 12.9%.

In a manner similar to the procedure described above, Compound XXIV was obtained in 65 OD^{260 nm} units (about 264 nmols) showing the overall yield of about 11%, using as the starting material 2.4 µmols of 5′-O-dimethoxytrityl-2′-O-(tert-butyldimethylsilyl)inosine-bound CPG and repeating 19 cycles in the condensation cycle using 5′-O-dimethoxytrityl-3′-O-(tert-butyldimethylsilyl)inosine-2′-O-(H-phosphonate) (Compound XXII) together with pivaloyl chloride.

Compounds XXIII and XXIV were dissolved in D₂O containing 10 mM TEAB buffer (pH 7.5) and ³¹P-NMR was measaured (device for measurement: JEOL GX-400FT-NMR manufactured by JEOL Inc.). When trimethyl phosphate was used as the external standarad, Compound XXIV showed the characteristic signal alone at 53-54 ppm and Compound XXIV showed the characteritic signal alone at 52-55 ppm, to multiplet phosphorothioate.

Furthermore, Compounds XXV, XXVI, XXXIII, XXXIV and XXXV were produced in a manner similar to the procedure described above.

On the other hand, Compound XXX was produced by, in the production steps of Compound XXV, performing the acid treatment after completion of the final condensation cycle, condensing with monomethoxytrityl-hexanediol-H-phosphonate, then performing the acid treatment, finally condensing with cholesteryl-H-phosphonate and thereafter carrying out in a similar manner. Compound XXXI was also produced by finally condensing with stearyl-H-phosphonate and thereafter carrying out in a similar manner.

With respect to Compound XXXII, the compound was produced by treating Compound XXV with acetic anhydride and pyridine. Compound XXXVI was produced using monomethoxytrityl-hexanediol-bound controlled pore glass as the starting material, by treating with an acid after completion of the condensation cycle as in the production of Compound XXV, then finally condensing with monomethoxytrityl-hexanediol-H-phosphonate and thereafter carrying out in a manner similar to the production of Compound XXV.

### Example 7 Test on anti-AIDS virus activity

Compound XIII which was a deoxyoligonucleotide (phosphate type) having base sequence complementary to the same regions as in Compound VI, Compound XI and Compound VI, Compound XIV which was a deoxyoligonucleotide (phosphate type) having base sequence complementary to the same region as in Compound XI and dCS (15-mer) which was deoxyoligocytidylate phosphorothioate, were dissolved in 10% fetal calf serum-containing RPMI 1640 medium in a definite concentration. MT-4 cells infected with AIDS virus (HTLV-III_{B} strain, 3 x 10⁵ PFU/ml) at a MOI of 0.002 were mixed with the aforesaid medium containing oligonucleotide in 3 x 10⁵/ml at the initial stage followed by culturing at 37°C. After the cell state was observed on Day 3, the system was again ajusted to cell counts of 3 x 10⁵ to 5 x 10⁵/ml. Oligonucleotide was also freshly supplemented to have the same concentration as in the initial stage. Cytopathic effect with viral infection was evaluated 6 days after the infection. The results are shown in Table 2, indicating that symbol + denotes the case in which viral infection and proliferation are inhibited and the cells grow normally and symbol - denotes the case in that the cells are destructed due to viral infection. In any case, no cytotoxicity was noted in tested concentrations.

**Table 2**

| | Concentration | | | | |
|---|---|---|---|---|---|
| Compound | 30 | 20 | 15 | 10 | 5 |
| Compound VI | | + | | + | - |
| Compound XIII (note 1) | | - | | - | |
| Compound XI | + | | - | | |
| Compound XIV (note 2) | | - | | - | |
| dCS (15-mer) (note 3) | | | | - | - |

| | | | | | |
|---|---|---|---|---|---|
| (Note 1) Compound XIII: deoxyoligonucleotide (phosphate type) complementary to the same region as in Compound VI. | | | | | |
| (Note 2) Compound XIV: deoxyoligonucleotide (phosphate type) complementary to the same region as in Compound XI. | | | | | |
| (Note 3) dCS (15-mer): deoxyoligocytidylate phosphorothioate (15-mer). | | | | | |

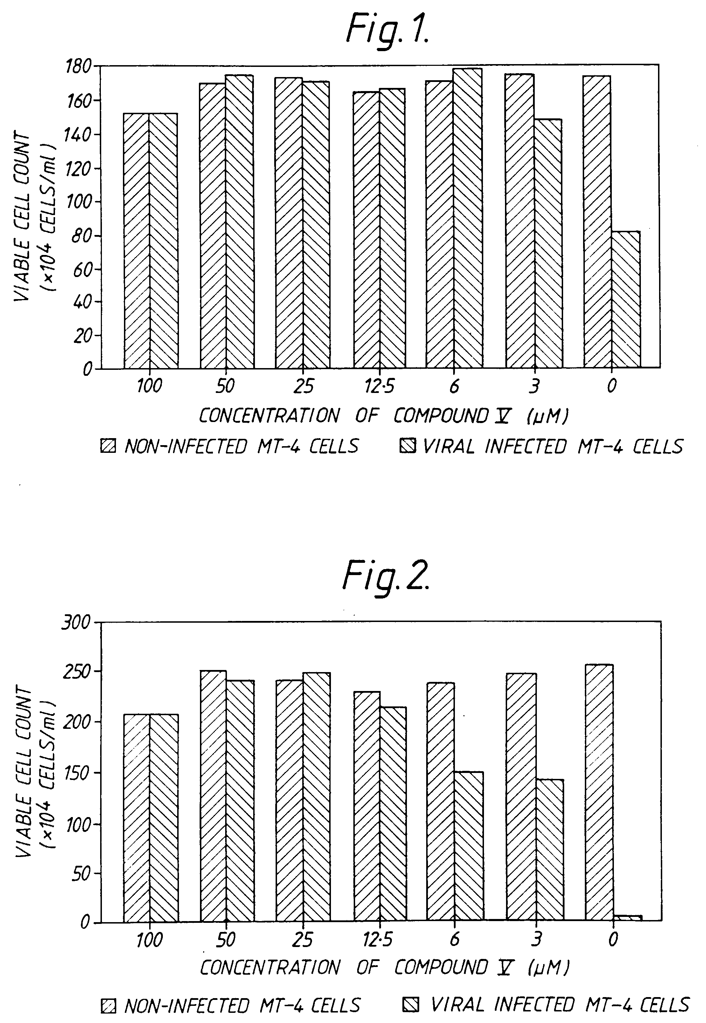

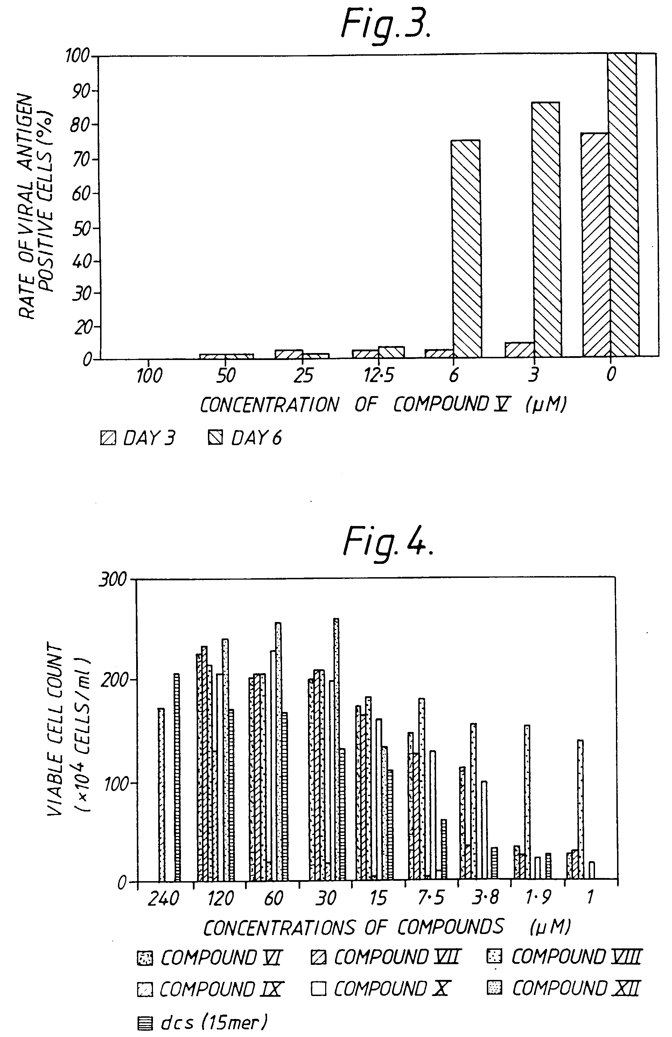

With respect to Compound VI, further detailed test was carried out. That is, Compound VI was dissolved in 10% fetal calf serum-containing RPMI 1640 medium in concentrations of 100 µM, 50 µM, 25 µM, 12.5 µM, 6 µM and 3 µM, at the time when the test was initiated. MT-4 cells infected with AIDS virus (HTLV-III_{B} strain) at a MOI of 0.002 were mixed with the medium containing Compound VI in 30 x 10⁴ cells/ml at the onset, followed by culturing in a CO₂ incubator at 37°C. For control, non-infected MT-4 cells were cultured in the medium containing Compound VI at the same time. A part of the culture was taken out on Day 3; viable cells were counted by trypan blue dye staining and a rate of viral antigen positive cells were determined by the indirect immunofluorescence method. On the other hand, in order to prevent influence on cell growth due to overgrowth, the culture cells were diluted again to 30 to 50 x 10⁴ cells/ml with the culture containing Compound VI in the same concentration and culture was further continued at 37°C. On Day 6 after the onset of test, the viable cell count and the rate of viral antigen positive cells were again determined. The results are shown in Table 3. The viable cell counts on Day 3 and Day 6 are shown in Figs. 1 and 2, respectively. The rate of viral antigen positive cells is shown in Fig. 3.

**Table 3**

| Anti-AIDS Viral Activity of Compound VI (viable cell count and rate of viral antigen positive cells) | | | | | | | | |
|---|---|---|---|---|---|---|---|---|
| Concentration of Compound VI (µM) | | 100 | 50 | 25 | 12.5 | 6 | 3 | 0 |
| Viable cell count on Day 3 (x 10⁴ cells/ml | Non-infected | 152 | 170 | 173 | 165 | 171 | 174 | 172 |
| | Infected | 153 | 175 | 171 | 166 | 178 | 147 | 79 |
| Viable cell count on Day 6 (x 10⁴ cells/ml | Non-infected | 207 | 249 | 239 | 228 | 236 | 245 | 252 |
| | Infected | 207 | 239 | 246 | 211 | 146 | 137 | 2 |
| Rate of viral antigen positive cells (%) | on Day 3 | 0 | 1 | 1.8 | 2.3 | 2.2 | 4 | 75 |
| | on Day 6 | 0 | 1 | 1 | 2.8 | 72 | 85 | 100 |

With respect to Compounds VI, VII, VIII, IX, X, XII and deoxyoligocytidylate phosphorothiate: dCS (15-mer), similar test was also carried out to determine the viable cell count and rate of viral antigen positive cells on Day 6 after infection.

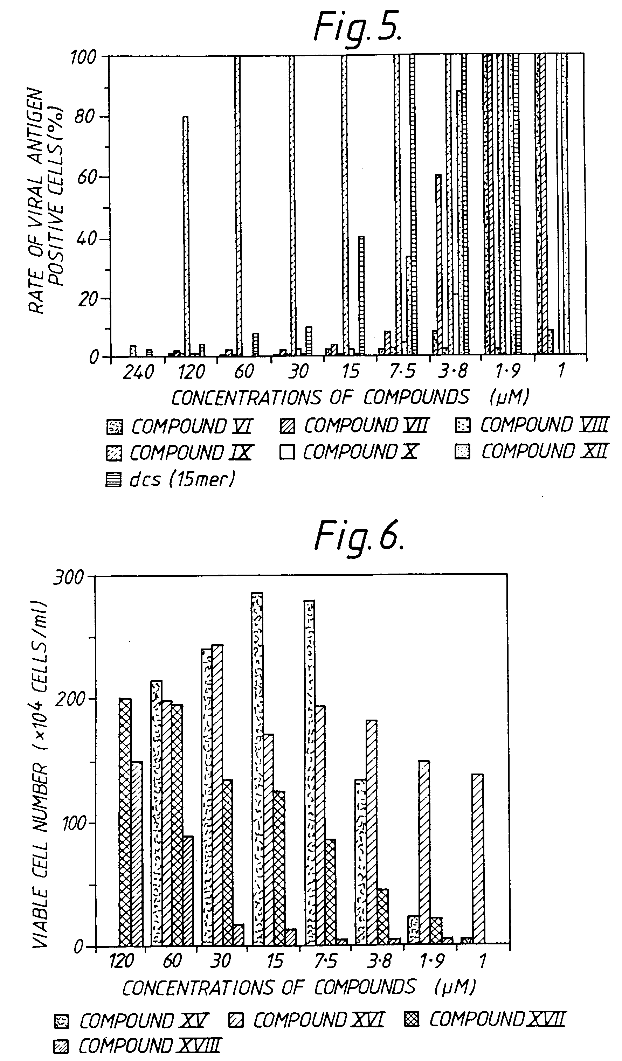

These results are shown in Table 4 with respect to the cell count on Day 6 after infection and in Table 5 with respect to the rate of viral antigen positive cells on Day 6 after infection. These results are also shown by graphs in Figs. 4 and 5, respectively.

**Table 4**

| Anti-AIDS Viral Activity of Compounds VI, VII, VIII, IX, X, XII and dCS (15-mer) (viable cell count on Day 6 after infection: x 10⁴ cells/ml) | | | | | | | | | |
|---|---|---|---|---|---|---|---|---|---|
| Concentration of Compound (µM) | 240 | 120 | 60 | 30 | 15 | 7.5 | 3.8 | 1.9 | 1 |
| Compound VI | | 225 | 201 | 199 | 171 | 145 | 110 | 31 | 24 |
| Compound VII | | 232 | 204 | 205 | 162 | 122 | 33 | 22 | 26 |
| Compound VIII | | 213 | 204 | 206 | 179 | 176 | 152 | 151 | 134 |
| Compound IX | 173 | 129 | 19 | 16 | 2 | 4 | 0 | 0 | |
| Compound X | | 205 | 226 | 196 | 157 | 124 | 96 | 20 | 15 |
| Compound XII | | 239 | 254 | 257 | 130 | 8 | 2 | 0 | 0 |
| dCS (15-mer) | 206 | 169 | 166 | 129 | 107 | 56 | 29 | 23 | |
| Non-infected MT-4 cells: 220 | | | | | | | | | |

**Table 5**

| Anti-AIDS Viral Activity of Compounds VI, VII, VIII, IX, X, XII and dCS (15-mer) (rate of viral antigen positive cells on Day 6 after infection: %) | | | | | | | | | |
|---|---|---|---|---|---|---|---|---|---|
| Concentration of Compound (µM) | 240 | 120 | 60 | 30 | 15 | 7.5 | 3.8 | 1.9 | 1 |
| Compound VI | | 1 | 1 | 1 | 2 | 2 | 8 | 100 | 100 |
| Compound VII | | 2 | 2 | 2 | 4 | 8 | 60 | 100 | 100 |
| Compound VIII | | 1 | 1 | 1 | 1 | 2 | 2 | 2 | 8 |
| Compound IX | 4 | 80 | 100 | 100 | 100 | 100 | 100 | 100 | |
| Compound X | | 1 | 1 | 2 | 2 | 4 | 20 | 100 | 100 |
| Compound XII | | 1 | 1 | 1 | 1 | 33 | 87 | 100 | 100 |
| dCS (15-mer) | 2 | 4 | 8 | 10 | 40 | 100 | 100 | 100 | |
| Non-infected MT-4 cells: 0 | | | | | | | | | |

With respect to Compounds XV, XVI, XVII, XVIII, XXIII and XXIV, similar test was also carried out. Regarding the results of Compounds XV, XVI, XVII and XVIII, the viable cell count on Day 6 after infection is shown in Table 6 and the rate of viral antigen positive cells on Day 6 after infection is shown in Table 7. These results are also shown by graphs in Figs. 6 and 7, respectively. On the other hand, with respect to the results of Compounds XXIII and XXIV, the viable cell count on Day 6 after infection is shown in Table 8 and the rate of viral antigen positive cells on Day 6 after infection is shown in Table 9. The viable cell counts of Compound XXIII and Compound XXIV are shown by graphs in Figs. 8 and 9, respectively. Furthermore, the rates of viral antigen positive cells of Compounds XXIII and XXIV on Day 6 after infection are shown by graphs in Fig. 10.

**Table 6**

| Anti-AIDS Viral Activity of Compounds XV, XVI, XVII and XVIII (viable cell count on Day 6 after infection: x 10⁴ cells/ml) | | | | | | | | |
|---|---|---|---|---|---|---|---|---|
| Concentration of Compound (µM) | 120 | 60 | 30 | 15 | 7.5 | 3.8 | 1.9 | 1 |
| Compound XV | | 215 | 240 | 285 | 277 | 135 | 21 | 3 |
| Compound XVI | | 198 | 242 | 171 | 192 | 181 | 148 | 137 |
| Compound XVII | 201 | 195 | 134 | 125 | 85 | 43 | 20 | |
| Compound XVIII | 151 | 89 | 16 | 13 | 3 | 1 | 2 | |
| Non-infected MT-4 cells: 220 | | | | | | | | |

**Table 7**

| Anti-AIDS Viral Activity of Compounds XV, XVI, XVII and XVIII (rate of viral antigen positive cells on Day 6 after infection: %) | | | | | | | | |
|---|---|---|---|---|---|---|---|---|
| Concentration of Compound (µM) | 120 | 60 | 30 | 15 | 7.5 | 3.8 | 1.9 | 1 |
| Compound XV | | 1 | 1 | 1 | 2 | 78 | 99 | 99 |
| Compound XVI | | 1 | 1 | 1 | 1 | 1 | 2 | 5 |
| Compound XVII | 1 | 1 | 2 | 4 | 80 | 100 | 100 | |
| Compound XVIII | 1 | 80 | 100 | 100 | 100 | 100 | 100 | |
| Non-infected MT-4 cells: 0 | | | | | | | | |

**Table 8**

| Anti-AIDS Viral Activity of Compounds XXIII and XXIV (viable cell count on Day 6 after infection: x 10⁴ cells/ml) | | | | | | | |
|---|---|---|---|---|---|---|---|
| Concentration of Compound (µM) | 60 | 30 | 15 | 7.5 | 3.8 | 1.9 | 0 |
| Compound XXIII: | | | | | | | |
| Non-infected | 147 | 141 | 161 | 152 | 138 | 133 | 161 |
| Infected | 159 | 159 | 156 | 142 | 127 | 10 | 2 |

| Compound XXIV: | | | | | | | |
|---|---|---|---|---|---|---|---|
| Non-infected | 149 | 160 | 142 | 155 | 159 | 143 | 161 |
| Infected | 149 | 173 | 157 | 153 | 11 | 3 | 2 |

**Table 9**

| Anti-AIDS Viral Activity of Compounds XXIII and XXIV (rate of viral antigen positive cells on Day 6 after infection: %) | | | | | | | |
|---|---|---|---|---|---|---|---|
| Concentration of Compound (µM) | 60 | 30 | 15 | 7.5 | 3.8 | 1.9 | 0 |
| Compound XXIII | 0.2 | 0.2 | 0.2 | 0.2 | 41 | >90 | >90 |
| Compound XXIV | 0.2 | 0.2 | 4.3 | >90 | ->90 | >90 | >90 |

As described above, the novel 2'-O-methylribooligonucleotide phosphorothioate derivatives and the novel ribooligonucleotide phosphorothioate derivatives according to the present invention have anti-AIDS viral activity and are expected to be used as drugs such as drugs for treatment of AIDS. Therefore, the present invention is extremely useful in the drug industries.

Reference is made above to the accompanying drawings in which:
Figs. 1 and 2 show the measurement results on the viable cell count of Compound VI on Day 3 and Day 6, respectively;
Fig. 3 shows the measurement results on the rate of viral antigen positive cells;
Figs. 4 and 5 show the measurement results on the viable cell count and the rate of viral antigen positive cells of Compounds VI, XII, VIII, IX, X, XII and deoxyoligocytidylate phosphorothioate: dCS (15-mer) on Day 6 after infection, respectively;

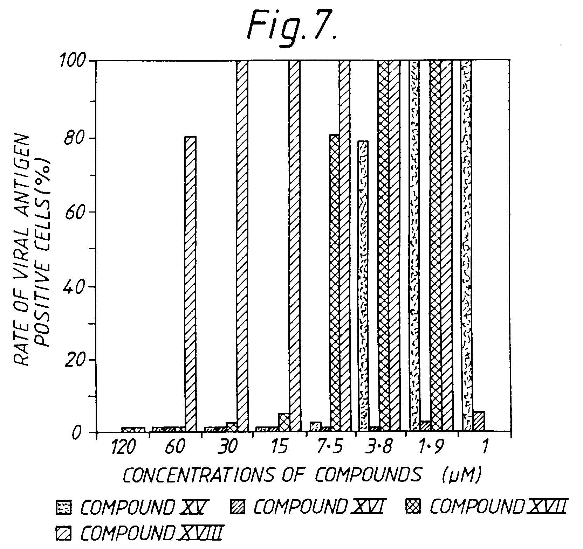

Figs. 6 and 7 show the measurement results, of Example 7, on viable cell count and rate of viral antigen positive cells of Compounds XV, XVI, XVII and XVIII on Day 6 after infection, respectively;

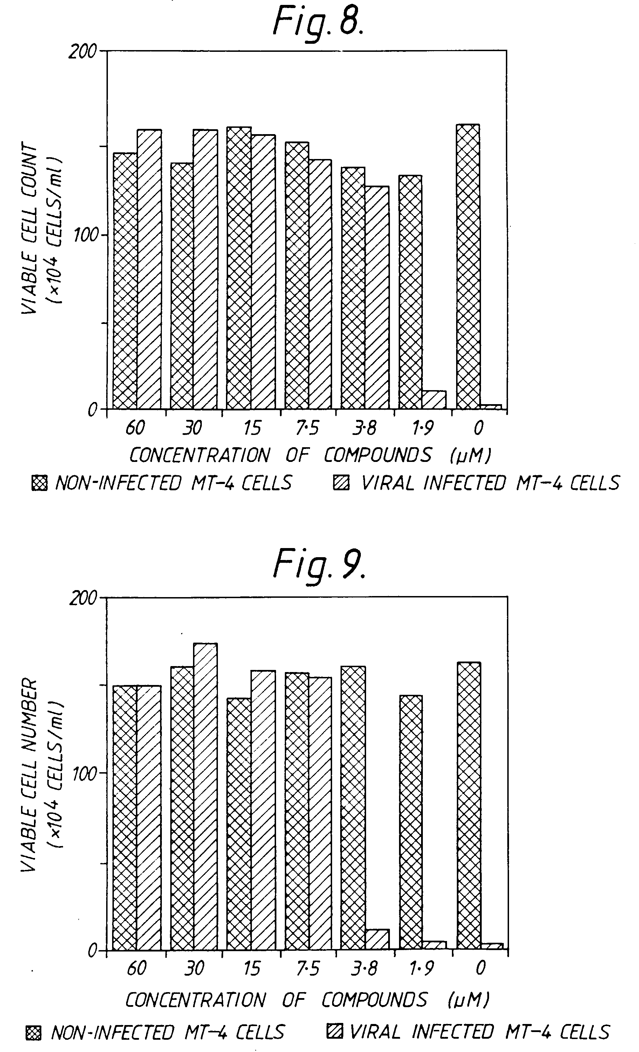

Figs. 8 and 9 show the measurement results in Example 7 on viable cell count of Compounds XXIII and XXIV on Day 6 after infection, respectively; and

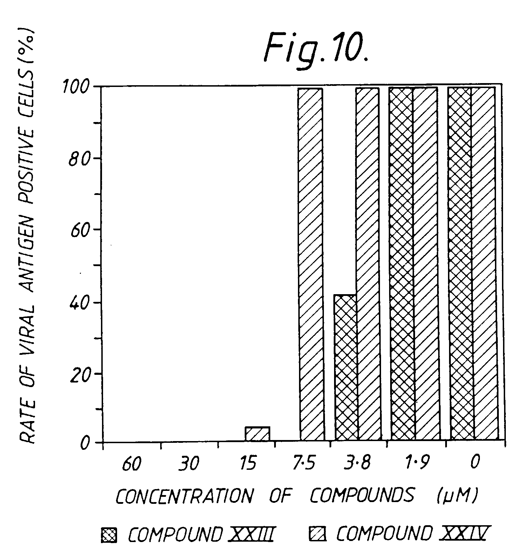

Fig. 10 shows the rate of viral antigen positive cells of Compounds XXIII and XXIV on Day 6 after infection, of Example 7.

## Claims

1. An oligoribonucleotide derivative represented by the general formula: 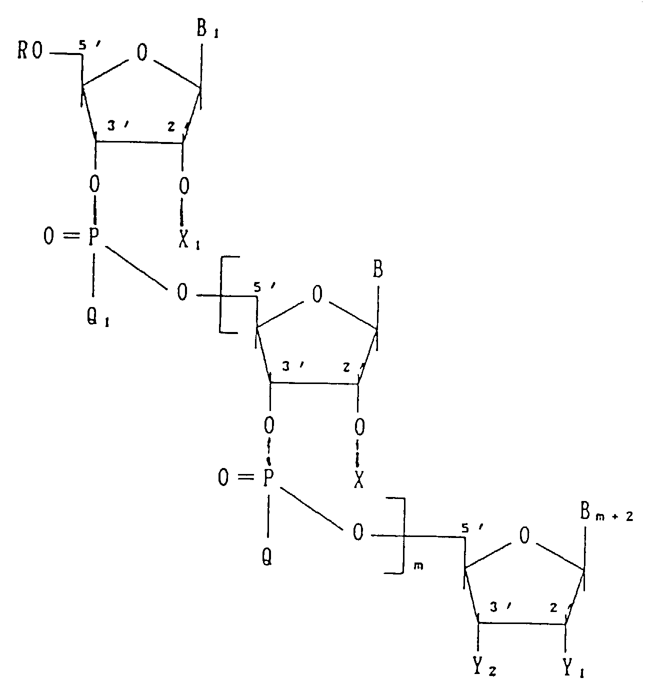 wherein:
m is an integer of 1 to 100;
B represents B₂, B₃ ..... B₍ₘ₊₁₎, and B₁ to B₍ₘ₊₂₎, which may be the same or different, each represents any one of hypoxanthine-9-yl, guanine-9-yl, adenine-9-yl, cytosine-1-yl, uracil-1-yl and thymine-1-yl;
Q represents Q₂, Q₃, ...... Q₍ₘ₊₁₎, and and Q₁ to Q₍ₘ₊₁₎, independently represents any one of a thioanion, an oxoanion, an alkyl group, an aryloxy group, an alkylamino group, an arylamino group, an alkylthio group and an arylthio group (provided that at least one of Q₁ to Q₍ₘ₊₁₎ represents a thioanion);
X represents X₂, X₃ ..... X₍ₘ₊₁₎, and X₁ to X₍ₘ₊₁₎, which may be the same or different, each represents any one of a hydrogen atom, a methyl group or an acyl group having 1 to 20 carbon atoms (provided that all of them do not represent a hydrogen atom);
R represents a hydrogen atom, an acyl group having 1 to 20 carbon atoms, a phosphoryl group which may optionally have a substituent, a thiophosphoryl group which may optionally have a substituent or an alkylaminophosphoryl group which may optionally have a substituent; and
Y₁ and Y₂ independently represent a methoxy group, a acyloxy group having 1 to 20 carbon atoms, a hydrogen atom, a hydroxyl group, an alkyloxy group which may optionally have a substituent, a phosphoryloxy group which may optionally have a substituent, a thiophosphoryloxy group which may optionally have a substituent and an alkylaminophosphoryloxy group which may optionally have a substituent.

2. A derivative as claimed in claim 1, wherein said substituent on R, Y₁ and Y₂, which may be the same or different, each represents any one of a cyanoethyl group, a chlorophenyl group, a monophosphoryl group, a pyrophosphoryl group, a hydrocarbon residue having 1 to 20 carbon atoms, a cholesteryl group and a group having the formula J-(K-O-L)_{E} wherein J represents: 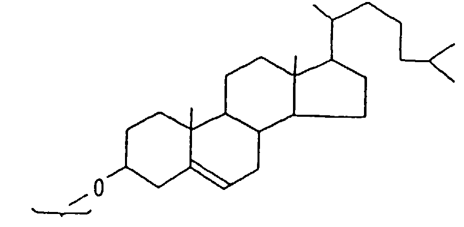 a hydroxyl group or any one of the oligoribonucleotidyl groups defined for the structural formula of said derivative according to claim 1, from which any one of RO, Y₁ and Y₂ is removed;
K represents any one of a phosphoryl group, a thiophosphoryl group and an alkylaminophosphoryl group having 1 to 10 carbon atoms;
L represents a hydrocarbon residue having 1 to 20 carbon atoms; and
E represents an integer of 1 to 10.

3. A derivative as claimed in claim 1, wherein m represents 26, B₁ to B₂₈ represents, in sequence, U, U, C, C, C, U, U, U, C, G, C, U, U, U, C, A, A, G, U, C, C, C, U, G, U, U, C and G; Q₁ to Q₂₇ each represent a thioanion; Y₁ and Y₂ represent OCH₃; R represents H; and X represents H (provided that all of them do not represent a hydrogen atom).

4. A derivative as claimed in claim 1, wherein m represents 26, B₁ to B₂₈ represent, in sequence U, C, C, U, U, C, U, A, G, C, U, C, C, G, C, U, A, G, U, C, A, A, A, A, U, U, U and U; Q₁ to Q₂₇ each represent a thioanion; Y₁ and Y₂ represent OCH₃; R represents H; and X represents H (provided that all of them do not represent a hydrogen atom).

5. A derivative as claimed in claim 1, wherein m represents 26, B₁ to B₂₈ represent, in sequence, U, U, U, U, A, A, U, U, U, A, U, A, U, U, U, U, U, U, C, U, U, U, C, C, C, C, C and U; Q₁ to Q₂₇ each represent a thioanion; Y₁ and Y₂ represent OCH₃; R represents H; and X represents H (provided that all of them do not represent a hydrogen atom).

6. A derivative as claimed in claim 1, having the structural formula:

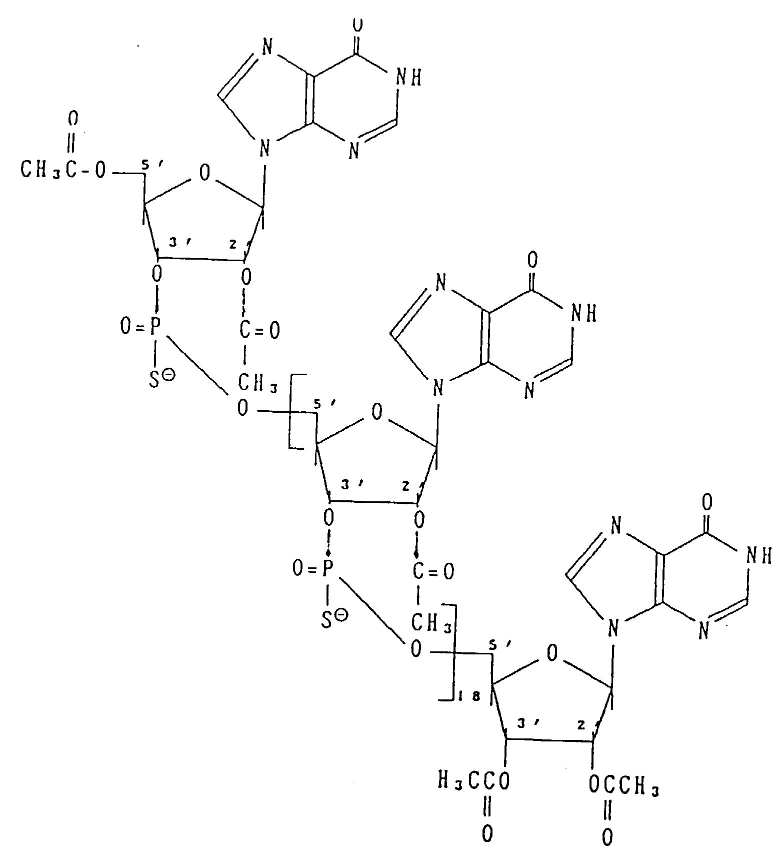

7. An anti-AIDS viral agent comprising as an effective ingredient at least oligoribonucleolide derivative as claimed in any of claims 1 to 6.

8. A 2'-O-methylribooligonucleotide phosphorothiate derivative containing a sequence complementary to genomic RNA of AIDS virus or to DNA integrated into a chromosome and represented by the general formula: 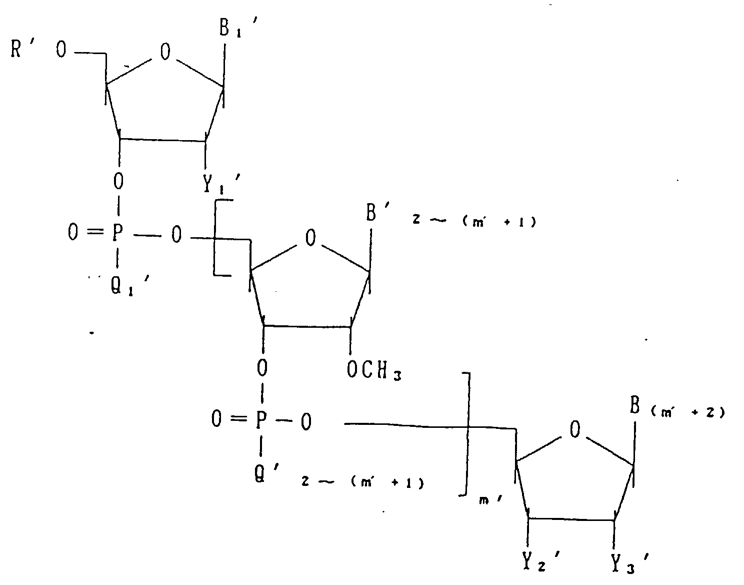 wherein:
m' is an integer of 1 to 50;
B'₁ to B'_{(m'+2)'} which may be the same or different, each represents any one of adenine-9-yl, guanine-9-yl, cytosine-1-yl, uracil-1-yl, thymine-1-yl and hypoxanthine-9-yl;
Q'₁ represents any one of a thioanion, an oxoanion, an alkyl group, an alkyloxy group, an aryloxy group, an alkylamino group, an arylamino group, an alkylthio group and an arylthio group;
at least one of Q'₂ to Q'_{(m'+1}), represents a thioanion and the other(s) is(are) either a thioanion or an oxoanion;
Y'₁ to Y'₃ independently represents a methoxy group, an alkyloxy group which may optionally have a substituent, a hydroxyl group, an alkylsilyl group or a hydrogen atom;
R' represents a hydrogen atom, a phosphoryl group or a thiophosphoryl group which may optionally have a substituent.

9. A derivative as claimed in claim 8, wherein m' represents 18; B'₁ to B'₂₀ represent, in sequence, A, C, A, C, C, C, A, A, U, U, C, U, G, A, A, A, A, U, G and G; Q'₁ to Q'₁₉ represent a thioanion; Y'₁ andY'₂ represent OCH₃; Y'₃ represents OH; and R' represents a hydrogen atom.

10. A derivative as claimed in claim 8, wherein m' represents 15; B'₁ to B'₁₇ represent, in sequence, C, C, C, A, A, U, U, C, U, G, A, A, A, A, U, G and G; Q'₁ to Q'₁₆ represent a thioanion; Y'₁ and Y'₂ represent OCH₃; Y'₃ represents OH; and R' represents a hydrogen atom.

11. A derivative as claimed in claim 8, wherein m' represents 23; B'₁ to B'₂₅ represent, in sequence, A, C, A, C, C, C, A, A, U, U, C, U, G, A, A, A, A, U, G, G, A, U, A, A and A; Q'₁ to Q'₂₄ represent a thioanion; Y'₁ and Y'₂ represent OCH₃; Y'₃ represents OH; and R' represents a hydrogen atom.

12. A derivative as claimed in claim 8, wherein m' represents 8; B'₁ to B'₁₀ represent, in sequence, C, A, A, U, U, C, U, G, A and A; Q'₁ to Q'₉ represent a thioanion; Y'₁ and Y'₂ represent OCH₃; Y'₃ represents OH; and R' represents a hydrogen atom.

13. A derivative as claimed in claim 8, wherein m' represents 19; B'₁ to B'₂₁ represent, in sequence, G, U, C, C, C, U, G, U, U, C, G, G, G, C, G, C, C, A, C, U and G; Q'₁ to Q'₂₀ represent a thioanion; Y'₁ and Y'₂ represent OCH₃; Y'₃ represents OH; and R' represents a hydrogen atom.

14. A derivative as claimed in claim 8, wherein m' represents 19; B'₁ to B'₂₁ represent, in sequence, G, A, G, C, U, C, C, C, A, G, G, C, U, C, A, G, A, U, C, U and G; Q'₁ to Q'₂₀ represent a thioanion; Y'₁ and Y'₂ represent OCH₃; Y'₃ represents OH; and R' represents a hydrogen atom.

15. A derivative as claimed in claim 8, wherein m' represents 18; B'₁ to B'₂₀ represent, in sequence, A, C, A, C, C, C, A, A, U, U, C, U, G, A, A, A, A, U, G, and G; Q'₁ to Q'₅ represent a thioanion; Q'₆ to Q'₁₄ represent an oxoanion; at least two of Q'₁₅ to Q'₁₉ represent a thioanion and the other three each represent an oxoanion; Y'₁ and Y'₂ represent OCH₃; Y'₃ represents OH; and R' represents a hydrogen atom.

16. A 2'-O-methylribooligonucleotide phosphorothioate derivative represented by the general formula: 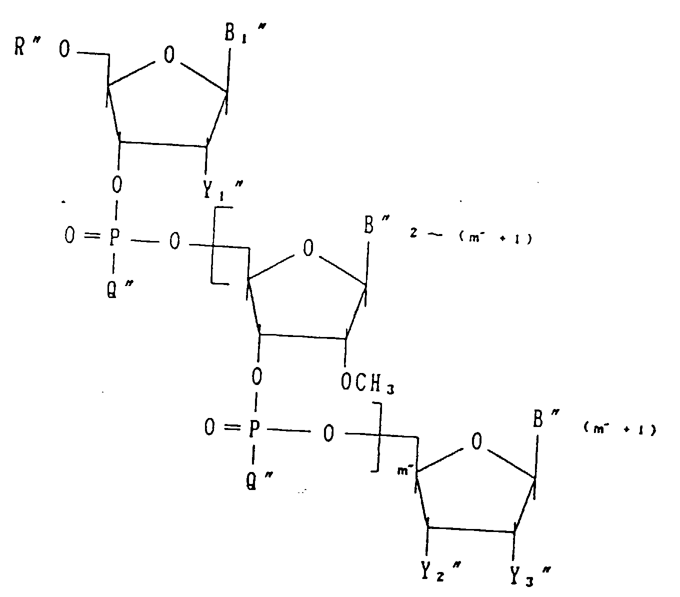 wherein:
m" is an integer of 1 to 50;
B"₁ to B"_{(m"+2)}, which may be the same or different, each represents any one of adenine-9-yl, guanine-9-yl, cytosine-1-yl, uracil-1-yl, thymine-1-yl and hypoxanthine-9-yl;
Q" contains at least one thioanion and represents any one of a thioanion, an oxoanion, an aryloxy group, an alkylamino group, an arylamino group, an alkylthio group and an arylthio group;
Y"₁ to Y"₃ independently represents a methoxy group, an alkyloxy group which may optionally have a substituent, a hydroxyl group, an alkylsilyl group and a hydrogen atom;
R" represents a hydrogen atom or a phosphoryl group which may optionally have a substituent.

17. A derivative as claimed in claim 16, wherein m" represents 18; B"₁ to B"₂₀ represents adenine-9-yl; Q" represents a thioanion; Y"₁ and Y"₂ represent OCH₃; Y"₃ represents OH; and R" represents a hydrogen atom.

18. A derivative as claimed in claim 16, wherein m" represents 18; B"₁ to B"₂₀ represent hypoxanthine-9-yl; Q" represents a thioanion; Y"₁ and Y"₂ represent OCH₃; Y"₃ represents OH; and R" represents a hydrogen atom.

19. A derivative as claimed in claim 16, wherein m" represents 18; B"₁ to B"₂₀ represent uracil-1-yl; Q" represents a thioanion; Y"₁ and Y"₂ represent OCH₃; Y"₃ represents OH; and R" represents a hydrogen atom.

20. A derivative as claimed in claim 16, wherein m" represents 18; B"₁ to B"₂₀ represent cytosine-1-yl; Q" represents a thioanion; Y"₁ and Y"₂ represent OCH₃; Y"₃ represents OH; and R" represents a hydrogen atom.

21. An oligoribonucleotide derivative represented by the general formula: 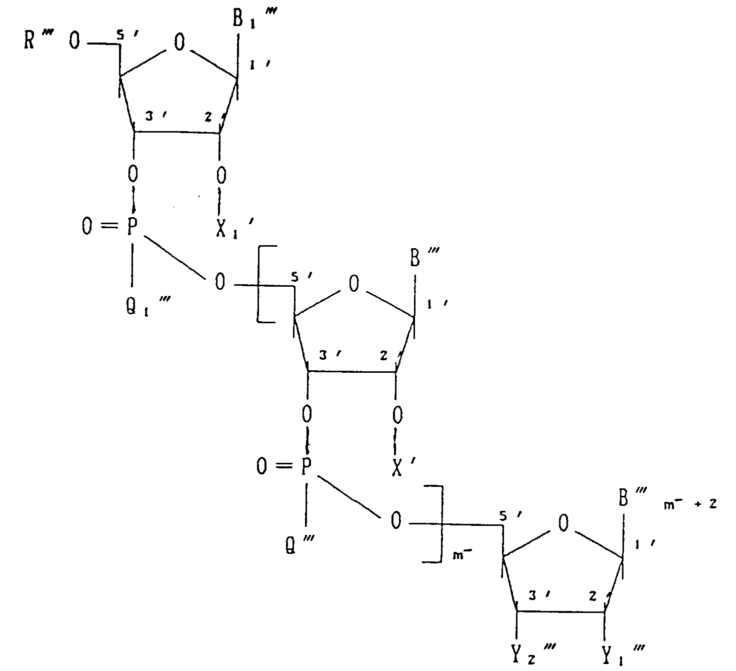 wherein:
m"' is an integer of 1 to 100;
B"' represents B"'₂, B"'₃, ..... B"'_{(m"'+1)}, and B"'₁ to B"'_{(m"'+2')} which may be the same or different, each represents any one of hypoxanthine-9-yl, guanine-9-yl, adenine-9-yl, cytosine-1-yl, uracil-1-yl and thymine-1-yl (provided that all of them do not represent adenine-9-yl);
Q''' represents Q"'₂, Q"'₃, ..... Q"'_{(m"'+1)}, and Q"'₁ to Q"'_{(m"'+1)}, independently represents any one of a thioanion, an oxoanion, an aryloxy group, an alkylamino group, an arylamino group, an alkylthio group and an arylthio group (provided that at least one of Q"'₁ to Q"'_{(m"'+1)}, represents a thioanion);
X' represents X'₂, X'₃ ..... X'_{(M"'+1)}, and X'₁ to X'_{(m"'+1)} each represents a hydrogen atom or methyl group (provided that all of them do not represent a methyl group and provided that all of them do not represent a hydrogen atom);
R"' represents any one of a hydrogen atom, an acyl group having 1 to 20 carbon atoms and a phosphoryl group which may optionally have a substituent; and
Y"'₁ and Y"'₂ independently represents any one of a methoxy group, an acyloxy group having 1 to 20 carbon atoms, an alkyloxy group which may optionally have a substituent, a hydroxyl group, an alkylsilyl group, a hydrogen atom and a phosphoryl group which may optionally have a substituent.

22. A derivative as claimed in claim 21, wherein said substituent is any one of a cyanoethyl group, a chlorophenyl group and a hydrocarbon residue having 1 to 20 carbon atoms.

23. A derivative as claimed in claim 21, having the structural formula:

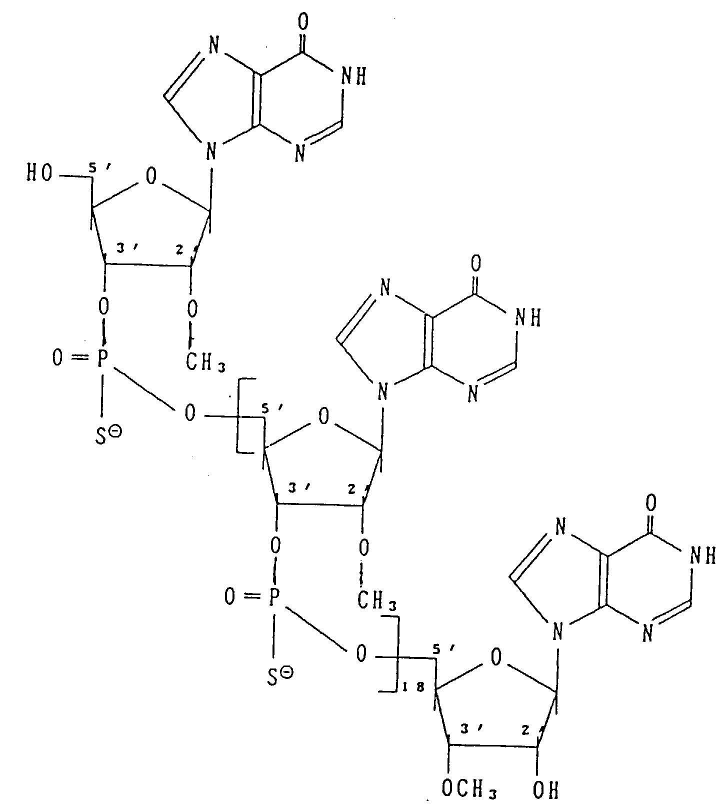

## Patentansprüche

1. Oligoribonukleotidderivat, dargestellt durch die allgemeine Formel: 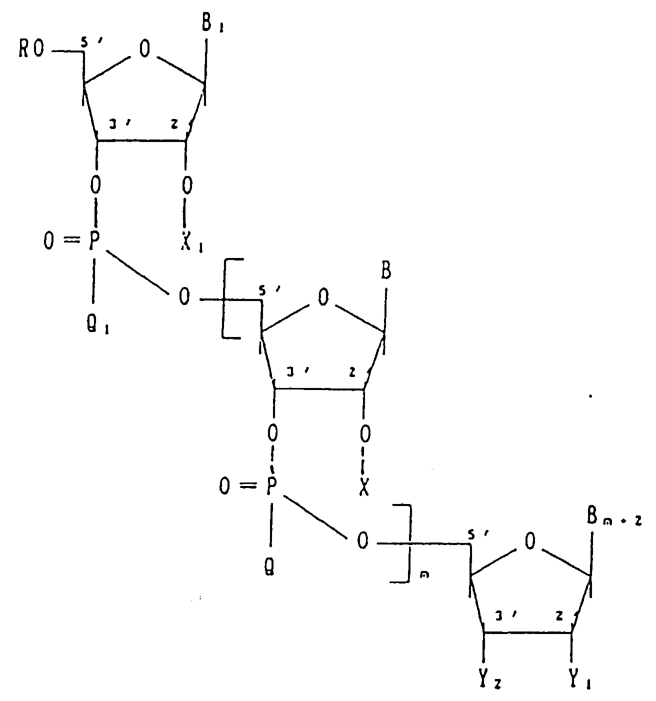 wobei:
m eine ganze Zahl von 1 bis 100 ist;
B B₂, B₃, ..... B₍ₘ₊₁₎ darstellt, und B₁ bis B₍ₘ₊₂₎ , die gleich oder verschieden sein können, jeweils einen der Reste Hypoxanthin-9-yl, Guanin-9-yl, Adenin-9-yl, Cytosin-1-yl, Uracil-1-yl bzw. Thymin-1-yl darstellen.
Q Q₂, Q₃, ..... Q₍ₘ₊₁₎ darstellt, und Q₁ bis Q₍ₘ₊₁₎ unabhängig voneinander ein Thioanion, ein Oxoanion, eine Alkylgruppe, eine Aryloxygruppe, eine Alkylaminogruppe, eine Arylaminogruppe, eine Alkylthiogruppe bzw. eine Arylthiogruppe darstellen (mit der Maßgabe, daß zumindest einer der Reste aus Q₁ bis Q₍ₘ₊₁) ein Thioanion darstellt);
X X₂, X₃ .... X₍ₘ₊₁₎ darstellt, und X₁ bis X₍ₘ₊₁₎, die gleich oder verschieden sein können, jeweils ein Wasserstoffatom, eine Methylgruppe oder eine Acylgruppe mit 1 bis 20 Kohlenstoffatomen darstellen (mit der Maßgabe, daß nicht alle von ihnen ein Wasserstoffatom darstellen);
R ein Wasserstoffatom, eine Acylgruppe mit 1 bis 20 Kohlenstoffatomen, eine Phosphorylgruppe, die gegebenenfalls einen Substituenten besitzen kann, eine Thiophosphorylgruppe, die gegebenenfalls einen Substituenten besitzen kann, oder eine Alkylaminophosphorylgruppe, die gegebenenfalls einen Substituenten besitzen kann, darstellt; und
Y₁ und Y₂ unabhängig voneinander eine Methoxygruppe, eine Acyloxygruppe mit 1 bis 20 Kohlenstoffatomen, ein Wasserstoffatom, eine Hydroxylgruppe, eine Alkyloxygruppe, die gegebenenfalls einen Substituenten haben kann, eine Phosphoryloxygruppe, die gegebenenfalls einen Substituenten haben kann, eine Thiophosphoryloxygruppe, die gegebenenfalls einen Substituenten haben kann, und eine Alkylaminophosphoryloxygruppe, die gegebenenfalls einen Substituenten haben kann, darstellen.

2. Derivat nach Anspruch 1, wobei der Substituent an R, Y₁ und Y₂, die gleich oder verschieden sein können, jeweils eine Cyanoethylgruppe, eine Chlorphenylgruppe, eine Monophosphorylgruppe, eine Pyrophosphorylgruppe, ein Kohlenwasserstoffrest mit 1 bis 20 Kohlenstoffatomen, eine Cholesterylgruppe bzw. eine Gruppe mit der Formel J-(K-O-L)_{E} darstellt, wobei J: 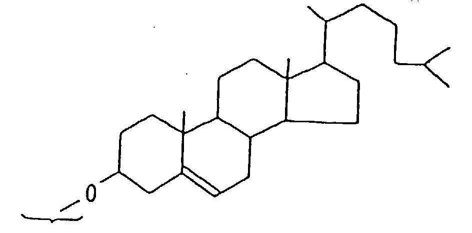
eine Hydroxylgruppe oder eine der Oligoribonukleotidylgruppen darstellt, die für die Strukturformel des Derivates nach Anspruch 1 definiert sind, aus denen einer der Reste RO, Y₁ bzw. Y₂ entfernt ist;
K eine Phosphorylgruppe, eine Thiophosphorylgruppe bzw. eine Alkylaminophosphorylgruppe mit 1 bis 10 Kohlenstoffatomen darstellt;
L einen Kohlenwasserstoffrest mit 1 bis 20 Kohlenstoffatomen darstellt; und
E eine ganze Zahl von 1 bis 10 darstellt.

3. Derivat nach Anspruch 1, wobei m 26 darstellt, B₁ bis B₂₈ in der Sequenz U, U, C, C, C, U, U, U, C, G, C, U, U, U, C, A, A, G, U, C, C, C, U, G, U, U, C und G darstellen; Q₁ bis Q₂₇ jeweils ein Thioanion darstellen; Y₁ und Y₂ OCH₃ darstellen; R H darstellt; und X H darstellt (mit der Maßgabe, daß nicht alle von ihnen ein Wasserstoffatom darstellen).

4. Derivat nach Anspruch 1, wobei m 26 darstellt, B₁ bis B₂₈ in der Sequenz U, C, C, U, U, C, U, A, G, C, U, C, C, G, C, U, A, G, U, C, A, A, A, A, U, U, U und U darstellen; Q₁ bis Q₂₇ jeweils ein Thioanion darstellen; Y₁ und Y₂ OCH₃ darstellen; R H darstellt und X H darstellt (mit der Maßgabe, daß nicht alle von ihnen ein Wasserstoffatom darstellen).

5. Derivat nach Anspruch 1, wobei m 26 darstellt, B₁ bis B₂₈ in der Sequenz U, U, U, U, A, A, U, U, U, A, U, A, U, U, U, U, U, U, C, U, U, U, C, C, C, C, C und U darstellen; Q₁ bis Q₂₇ jeweils ein Thioanion darstellen; Y₁ und Y₂ OCH₃ darstellen; R H darstellt und X H darstellt (mit der Maßgabe, daß nicht alle von ihnen ein Wasserstoffatom darstellen).

6. Derivat nach Anspruch 1 mit der Strukturformel:

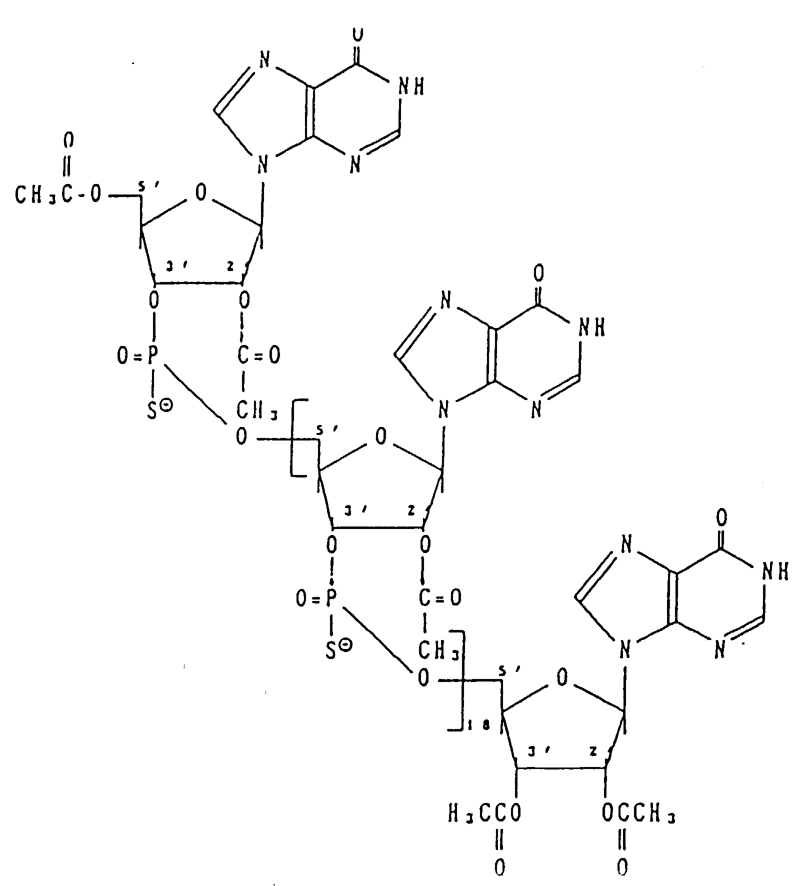

7. Anti-AIDS-Virusmittel, das als Wirkstoff zumindest das Oligoribonukleotidderivat nach einem der Ansprüche 1 bis 6 umfaßt.

8. 2'-O-Methylribooligonukleotidphosphorthioatderivat mit einer Sequenz, die zu genomischer RNA aus AIDS-Virus oder zu DNA, die in ein Chromosom integriert ist, komplementär ist, und dargestellt wird durch die allgemeine Formel: 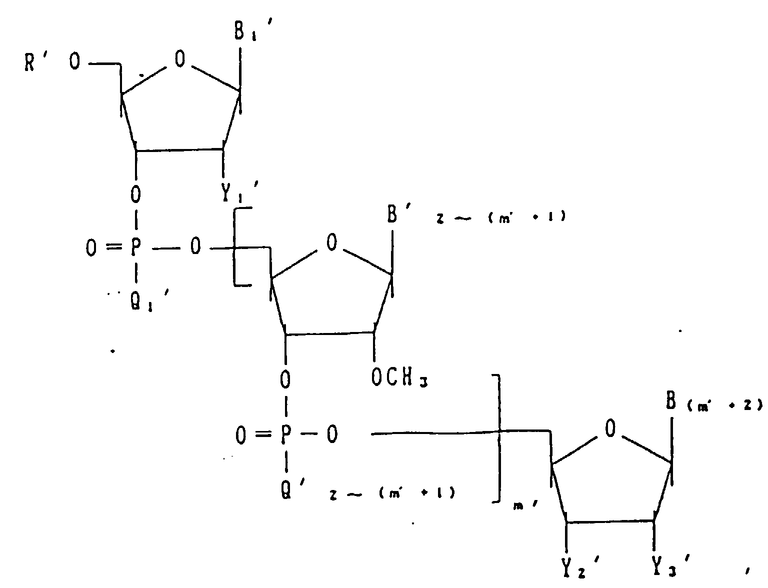 wobei:
m' eine ganze Zahl von 1 bis 50 ist;
B'₁ bis B'_{(m'+2)} , die gleich oder verschieden sein können, jeweils einen der Reste Adenin-9-yl, Guanin-9-yl, Cytosin-1-yl, Uracil-1-yl, Thymin-1-yl bzw. Hypoxanthin-9-yl darstellen.
Q'₁ ein Thioanion, ein Oxoanion, eine Alkylgruppe, eine Alkyloxygruppe, eine Aryloxygruppe, eine Alkylaminogruppe, eine Arylaminogruppe, eine Alkylthiogruppe bzw. eine Arylthiogruppe darstellt wobei zumindest einer der Reste Q'₂ bis Q'_{(m'+1}) ein Thioanion darstellt und der/die andere(n) entweder ein Thioanion oder ein Oxoanion ist/sind;
Y'₁ bis Y'₃ unabhängig voneinander eine Methoxygruppe, eine Alkyloxygruppe, die gegebenenfalls einen Substituenten haben kann, eine Hydroxylgruppe, eine Alkylsilylgruppe oder ein Wasserstoffatom darstellen;
R' ein Wasserstoffatom, eine Phosphorylgruppe oder eine Thiophosphorylgruppe, die gegebenenfalls einen Substituenten haben kann, darstellt.

9. Derivat nach Anspruch 8, wobei m' 18 ist; B'₁ bis B'₂₀ in der Sequenz A, C, A, C, C, C, A, A, U, U, C, U, G, A, A, A, A, U, G und G darstellen; Q'₁ bis Q'₁₉ ein Thioanion darstellen; Y'₁ und Y'₂ OCH₃ darstellen; Y'₃ OH darstellt; und R' ein Wasserstoffatom darstellt.

10. Derivat nach Anspruch 8, wobei m' 15 ist; B'₁ bis B'₁₇ in der Sequenz C, C, C, A, A, U, U, C, U, G, A, A, A, A, U, G und G darstellen; Q'₁ bis Q'₁₆ ein Thioanion darstellen; Y'₁ und Y'₂ OCH₃ darstellen; Y'₃ OH darstellt; und R' ein Wasserstoffatom darstellt.

11. Derivat nach Anspruch 8, wobei m' 23 darstellt; B'₁ bis B'₂₅ in der Sequenz A, C, A, C, C, C, A, A, U, U, C, U, G, A, A, A, A, U, G, G, A, U, A, A und A darstellen; Q'₁ bis Q'₂₄ ein Thioanion darstellen; Y'₁ und Y'₂ OCH₃ darstellen; Y'₃ OH darstellt; und R' ein Wasserstoffatom darstellt.

12. Derivat nach Anspruch 8, wobei m' 8 darstellt; B'₁ bis B'₁₀ in der Sequenz C, A, A, U, U, C, U, G, A und A darstellen; Q'₁ bis Q'₉ ein Thioanion darstellen; Y'₁ und Y'₂ OCH₃ darstellen; Y'₃ OH darstellt; und R' ein Wasserstoffatom darstellt.

13. Derivat nach Anspruch 8, wobei m' 19 darstellt; B'₁ bis B'₂₁in der Sequenz G, U, C, C, C, U, G, U, U, C, G, G, G, C, G, C, C, A, C, U und G darstellen; Q'₁ bis Q'₂₀ ein Thioanion darstellen; Y'₁ und Y'₂ OCH₃ darstellen; Y'₃ OH darstellt; und R' ein Wasserstoffatom darstellt.

14. Derivat nach Anspruch 8, wobei m' 19 darstellt; B'₁ bis B'₂₁ in der Sequenz G, A, G, C, U, C, C, C, A, G, G, C, U, C, A, G, A, U, C, U und G darstellen; Q'₁ bis Q'₂₀ ein Thioanion darstellen; Y'₁ und Y'₂ OCH₃ darstellen; Y'₃ OH darstellt; und R' ein Wasserstoffatom darstellt.

15. Derivat nach Anspruch 8, wobei m' 18 darstellt; B'₁ bis B'₂₀ in der Sequenz A, C, A, C, C, C, A, A, U, U, C, U, G, A, A, A, A, U, G und G darstellen; Q'₁ bis Q'₅ ein Thioanion darstellen; Q'₆ bis Q'₁₄ ein Oxoanion darstellen; zumindest zwei der Reste Q'₁₅ bis Q'₁₉ ein Thioanion und die restlichen drei jeweils ein Oxoanion darstellen; Y'₁ und Y'₂ OCH₃ darstellen; Y'₃ OH darstellt; und R' ein Wasserstoffatom darstellt.

16. 2'-O-Methylribooligonukleotidphosphorthioatderivat, dargestellt durch die allgemeine Formel: 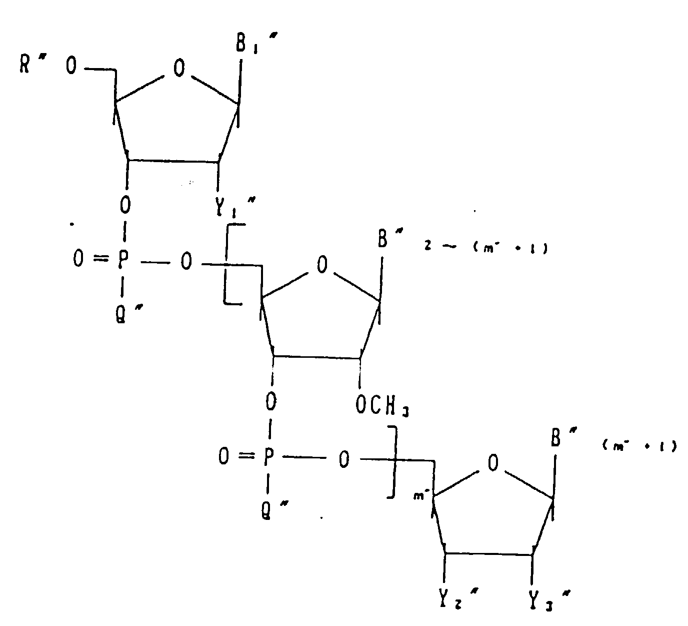 wobei:
m" eine ganze Zahl von 1 bis 50 ist;
B"₁ bis B"_{(m"+2)}, die gleich oder verschieden sein können, jeweils einen der Reste Adenin-9-yl, Guanin-9-yl, Cytosin-1-yl, Uracil-1-yl, Thymin-1-yl bzw. Hypoxanthin-9-yl darstellen;
Q" zumindest ein Thioanion enthält, und ein Thioanion, ein Oxoanion, eine Aryloxygruppe, eine Alkylaminogruppe, eine Arylaminogruppe, eine Alkylthiogruppe bzw. eine Arylthiogruppe darstellt; Y"₁ bis Y"₃ unabhängig voneinander eine Methoxygruppe, eine Alkyloxygruppe, die gegebenenfalls einen Substituenten haben kann, eine Hydroxylgruppe, eine Alkylsilylgruppe bzw. ein Wasserstoffatom darstellen;
R" ein Wasserstoffatom oder eine Phosphorylgruppe darstellt, die gegebenenfalls einen Substituenten haben kann.

17. Derivat nach Anspruch 16, wobei m" 18 darstellt; B"₁ bis B"₂₀ Adenin-9-yl darstellen; Q" ein Thioanion darstellt; Y"₁ und Y"₂ OCH₃ darstellen; Y"₃ OH darstellt; und R" ein Wasserstoffatom darstellt.

18. Derivat nach Anspruch 16, wobei m" 18 darstellt; B"₁ bis B"₂₀ Hypoxanthin-9-yl darstellen; Q" ein Thioanion darstellt; Y"₁ und Y"₂ OCH₃ darstellen; Y"₃ OH darstellt; und R" ein Wasserstoffatom darstellt.

19. Derivat nach Anspruch 16, wobei m" 18 darstellt; B"₁ bis B"₂₀ Uracil-1-yl darstellen; Q" ein Thioanion darstellt; Y"₁ und Y"₂ OCH₃ darstellen; Y"₃ OH darstellt; und R" ein Wasserstoffatom darstellt.

20. Derivat nach Anspruch 16, wobei m" 18 darstellt; B"₁ bis B"₂₀ Cytosin-1-yl darstellen; Q" ein Thioanion darstellt; Y"₁ und Y"₂ OCH₃ darstellen; Y"₃ OH darstellt; und R" ein Wasserstoffatom darstellt.

21. Oligoribonukleotidderivat, dargestellt durch die allgemeine Formel: 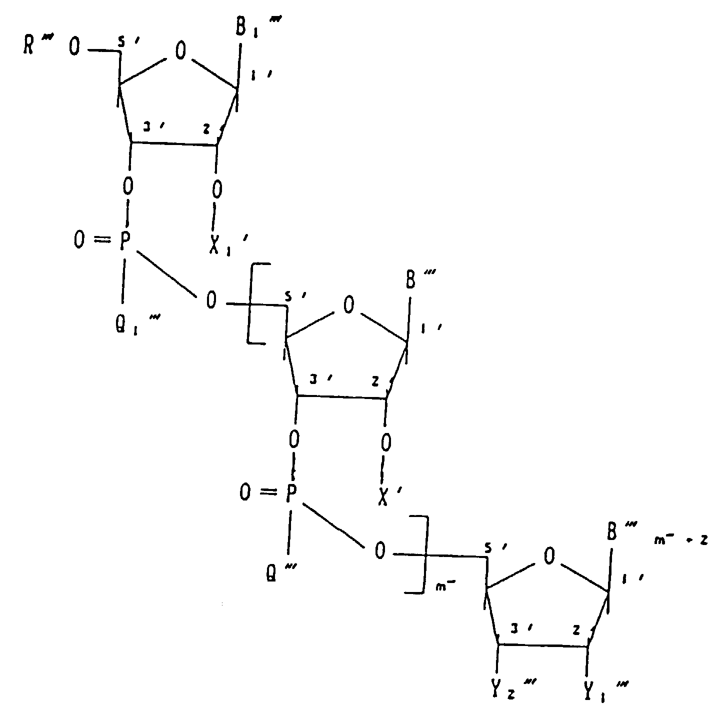 wobei:
m"' eine ganze Zahl von 1 bis 100 ist;
B"' B"'₂, B"'₃, ..... B"'_{(m"'+1)} darstellt, und B"'₁ bis B"'_{(m"'+2)}, die gleich oder verschieden sein können, jeweils einen der Reste Hypoxanthin-9-yl, Guanin-9-yl, Adenin-9-yl, Cytosin-1-yl, Uracil-1-yl bzw. Thymin-1-yl darstellen (mit der Maßgabe, daß nicht alle von ihnen Adenin-9-yl darstellen);
Q"' Q"'₂, Q"'₃, ..... Q"'_{(m"'+1)} darstellt, und Q"'₁ bis Q"'_{(m"'+1)} unabhängig voneinander ein Thioanion, ein Oxoanion, eine Aryloxygruppe, eine Alkylaminogruppe, eine Arylaminogruppe, eine Alkylthiogruppe bzw. eine Arylthiogruppe darstellen (mit der Maßgabe, daß zumindest einer der Reste aus Q"'₁ bis Q"'_{(m"'+1)} ein Thioanion darstellt);
X' X'₂, X'₃, ..... X'_{(m"'+1)} darstellt, und X'₁ bis X'_{(m"'+1)} jeweils ein Wasserstoffatom oder eine Methylgruppe darstellen (mit der Maßgabe, daß nicht alle von ihnen eine Methylgruppe darstellen und mit der Maßgabe, daß nicht alle von ihnen ein Wasserstoffatom darstellen);
R"' ein Wasserstoffatom, eine Acylgruppe mit 1 bis 20 Kohlenstoffatomen und eine Phosphorylgruppe, die gegebenenfalls einen Substituenten besitzen kann, darstellt; und
Y"'₁ und Y"'₂ unabhängig voneinander eine Methoxygruppe, eine Acyloxygruppe mit 1 bis 20 Kohlenstoffatomen, eine Alkyloxygruppe, die gegebenenfalls einen Substituenten besitzen kann, eine Hydroxylgruppe, eine Alkylsilylgruppe, ein Wasserstoffatom, bzw. eine Phosphorylgruppe, die gegebenenfalls einen Substituenten haben kann, darstellen.

22. Derivat nach Anspruch 21, wobei der Substituent eine Cyanoethylgruppe, eine Chlorphenylgruppe, bzw. ein Kohlenwasserstoffrest mit 1 bis 20 Kohlenstoffatomen, ist.

23. Derivat nach Anspruch 21 mit der Strukturformel:

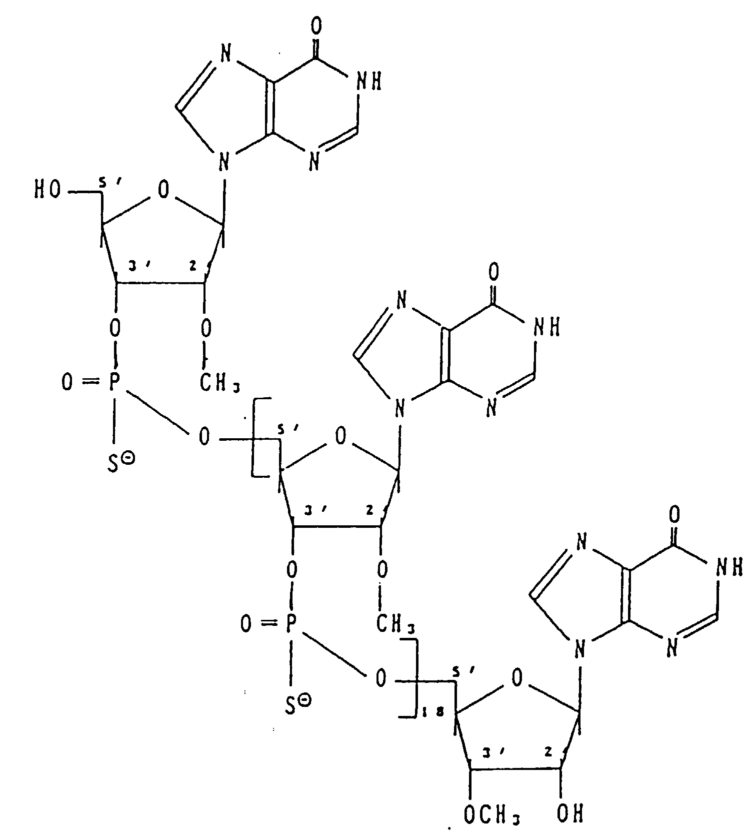

## Revendications

1. Dérivé d'oligoribonucléotide représenté par la formule générale : 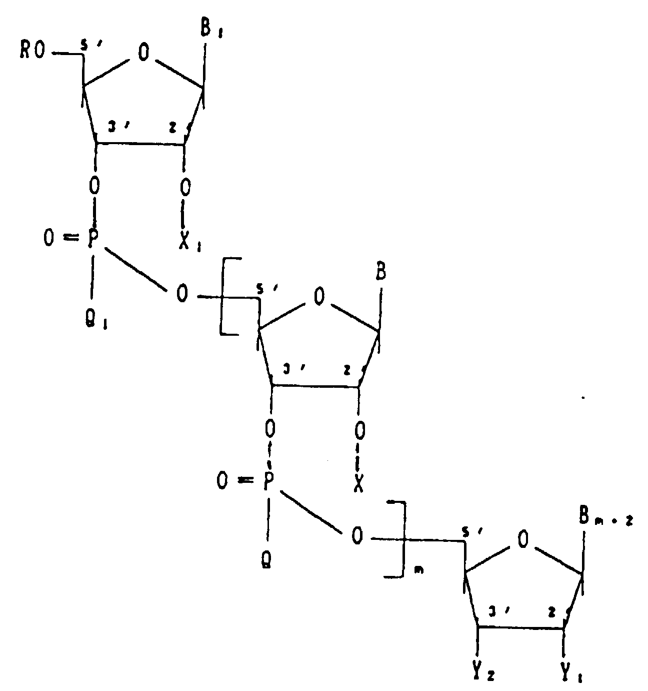 dans laquelle :
m est un nombre entier compris entre 1 et 100,
B représente B₂, B₃,... B₍ₘ₊₁₎ et, B₁ à B₍ₘ₊₂₎, qui peuvent être identiques ou différents, représentent chacun l'un quelconque parmi l'hypoxanthine-9-yle, la guanine-9-yle, l'adénine-9-yle, la cytosine-1-yle, l'uracil-1-yle et la thymine-1-yle,
Q représente Q₂, Q₃..... Q₍ₘ₊₁₎, et Q₁ à Q₍ₘ₊₁₎ représentent indépendamment l'un quelconque parmi un anion thio, un anion oxo, un groupe alkyle, un groupe aryloxy, un groupe alkylamino, un groupe arylamino, un groupe alkylthio et un groupe arylthio (à la condition qu'au moins un parmi Q₁ à Q₍ₘ₊₁₎ représente un anion thio),
X représente X₂, X₃ ..... X₍ₘ₊₁₎ et, X₁ à X₍ₘ₊₁₎, qui peuvent être identiques ou différents, représentent chacun l'un quelconque parmi un atome d'hydrogène, un groupe méthyle, ou un groupe acyle comportant de 1 à 20 atomes de carbone (à la condition qu'il ne représentent pas tous à la fois un atome d'hydrogène),
R représente un atome d'hydrogène, un groupe acyle comportant de 1 à 20 atomes de carbone, un groupe phosphoryle qui peut éventuellement porter un substituant, un groupe thiophosphoryle qui peut éventuellement porter un substituant ou un groupe alkylaminophosphoryle qui peut éventuellement porter un substituant, et
Y₁ et Y₂ représentent indépendamment un groupe métoxy, un groupe acyloxy comportant de 1 à 20 atomes de carbone, un atome d'hydrogène, un groupe hydroxy, un groupe alkyloxy qui peut éventuellement porter un substituant, un groupe phosphoryloxy qui peut éventuellement porter un substituant, un groupe thiophosphoryloxy qui peut éventuellement porter un substituant et un groupe alkylaminophosphoryloxy qui peut éventuellement porter un substituant.

2. Dérivé tel que revendiqué dans la revendication 1, dans lequel lesdits substituants sur R, Y₁ et Y₂, qui peuvent être identiques ou différents, représentent chacun l'un quelconque parmi un groupe cyanoéthyle, un groupe chlorophényle, un groupe monophosphoryle, un groupe pyrophosphoryle, un résidu hydrocarboné comportant de 1 à 20 atomes de carbones, un groupe cholestéryle et un groupe répondant à la formule J-(K-O-L)_{E} dans lequel J représente : 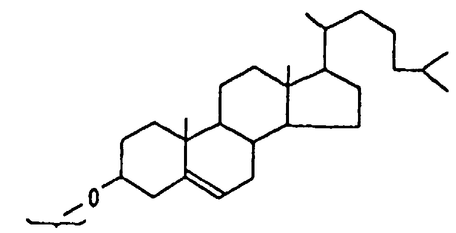
un groupe hydroxyle ou l'un quelconque des groupes oligoribonucléotidyles définis pour la formule de structure dudit dérivé selon la revendication 1, duquel on a enlevé l'un quelconque parmi RO, Y₁ et Y₂ ;
K représente l'un quelconque parmi un groupe phosphoryle, un groupe thiophosphoryle et un groupe alkylaminophosphoryle comportant de 1 à 20 atomes de carbone ;
L représente un résidu hydrocarboné comportant de 1 à 20 atomes de carbone ; et
E représenté un nombre entier compris entre 1 et 10.

3. Dérivé tel que revendiqué dans la revendication 1, dans lequel m représente 26, B₁ à B₂₈ représentent, en séquence, U, U, C, C, C, U, U, U, C, G, C, U, U, U, C, A, A, G, U, C, C, C, U, G, U, U, C et G, Q₁ à Q₇ représentent chacun un anion thio ; Y₁ et Y₂ représentent OCH₃ ; R représente H ; et X représente H (à la condition que tous ne représentent pas un atome d'hydrogène).

4. Dérivé tel que revendiqué dans la revendication 1, dans lequel m vaut 26, B₁ à B₂₈ représentent, en séquence, U, C, C, U, U, C, U, A,G, C, U, C, C, G, C, U, A, G, U, C, A, A, A, A, U, U, U et U, Q₁ à Q₂₇ représentent chacun un anion thio ; Y₁ et Y₂ représentent OCH₃ ; R représente H ; et X représente H (à la condition que tous ne représentent pas un atome d'hydrogène).

5. Dérivé tel que revendiqué dans la revendication 1, dans lequel M vaut 26, B₁ à B₂₈ représentent, en séquence, U, U, U, U, A, A, U, U, U, A, U, A, U, U, U, U, U, U, C, U, U, U, C, C, C, C, C, et U, Q₁ à Q₂₇ représentent chacun un anion thio ; Y₁ et Y₂ représentent OCH₃ ; R représente H ; et X représente H (à la condition que tous ne représentent pas un atome d'hydrogène).

6. Dérivé tel que revendiqué dans la revendication 1, ayant la formule de structure :

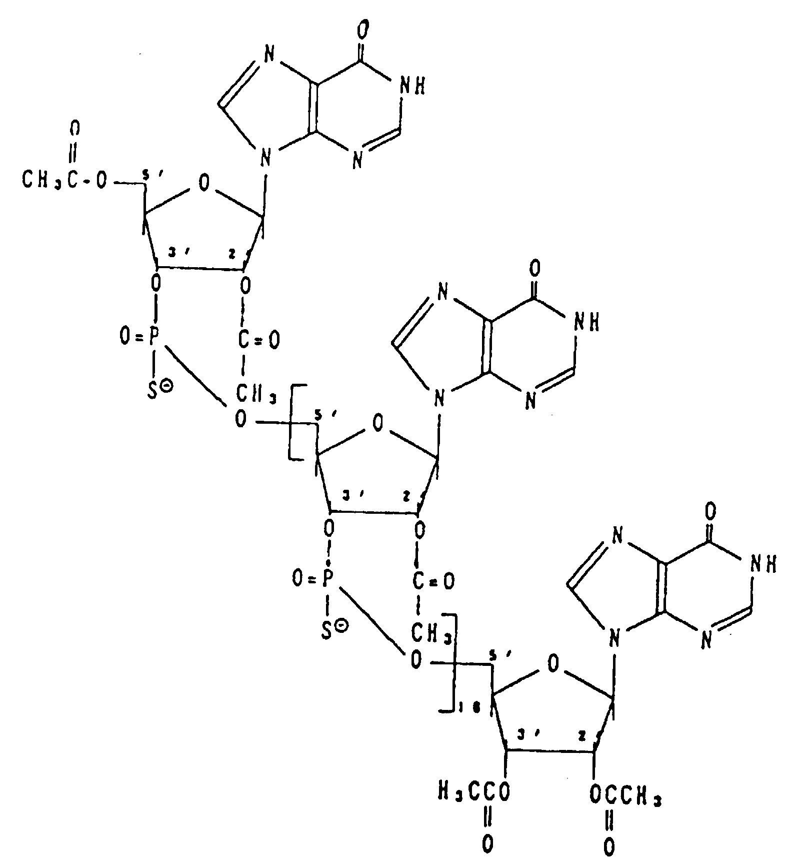

7. Agent anti-virus du SIDA, comprenant, comme ingrédient efficace, au moins un dérivé d'oligoribonucléotide revendiqué dans l'une quelconque des revendications 1 à 6.

8. Dérivé de 2'-O-méthylribooligonucléotide phosphorothioate contenant une séquence complémentaire de l'ARN génomique du virus du SIDA ou de l'ADN intégré dans un chromosome, et représenté par la formule générale : 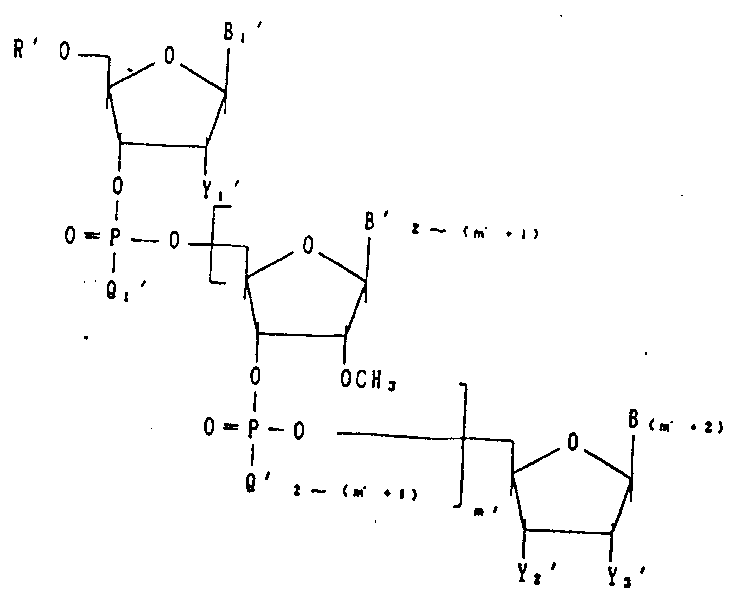 dans laquelle :
m' est un nombre entier compris entre 1 et 50 ;
B'₁ à B'_{(m'+2)}, qui peuvent être identiques ou différents, représentent chacun l'un quelconque parmi l'adénine-9-yle (A), la guanine-9-yle (G), la cytosine-1-yle (C), l'uracil-1-yle (U), la thymine-1-yle (T) et l'hypoxanthine-9-yle (H) ;
Q₁ représente l'un quelconque parmi un anion thio (S), un anion oxo (O⁻), un groupe alkyle, un groupe alkyloxy, un groupe aryloxy, un groupe alkylamino, un groupe arylamino, un groupe alkylthio et un groupe arylthio;
au moins un parmi Q'₂ à Q'_{(m'+1)} représente un anion thio (S⁻) et les autres représentent soit un anion thio soit un anion oxo (O⁻) ; Y'₁ à Y'₃ représentent indépendamment un groupe méthoxy, un groupe alkyloxy qui peut éventuellement porter un substituant, un groupe hydroxy, un groupe alkylsilyle et un atome d'hydrogène ;
R' représente un atome d'hydrogène, un groupe phosphoryle, ou un groupe thiophosphoryle qui peut éventuellement porter un substituant.

9. Dérivé tel que revendiqué dans la revendication 8, dans lequel m' vaut 18, B'₁ à B'₂₀ représentent, en séquence, A, C, A, C, C, C, A, A, U, U, C, U, G, A, A, A, A, U, G et G, Q'₁ à Q'₁₉ représentent un anion thio, Y_{'1} et Y'₂ représentent OCH₃ ; Y'₃ représente OH, et R' représente un atome d'hydrogène.

10. Dérivé tel que revendiqué dans la revendication 8, dans lequel m' vaut 15, B'₁ à B'₁₇ représentent, en séquence, C, C, C, A, A, U, U, C, U, G, A, A, A, A, U, G et G, Q'₁ à Q'₁₆ représentent un anion thio, Y'₁ et Y'₂ représentent OCH₃, Y'₃ représente OH, et R' représente un atome d'hydrogène.

11. Dérivé tel que revendiqué dans la revendication 8, dans lequel m' vaut 23, B'₁ à B'₂₅ représentent, en séquence, A, C, A, C, C, C,A,A,U, U, C, U, G, A, A, A, A, U, G, G, A, U, A, A et A, Q'₁ à Q'₂₄ représentent un anion thio, Y'₁ et Y'₂ représentent OCH₃, Y'₃ représente OH, et R' représente un atome d'hydrogène.

12. Dérivé tel que revendiqué dans la revendication 8, dans lequel m' vaut 8, B'₁ à B'₁₀ représentent, en séquence, C, A, A, U, U, C, U, G, A et A, Q'₁ à Q'₉ représentent un anion thio ; Y'₁ et Y'₂ représentent OCH₃, Y'₃ représente OH, et R' représente un atome d'hydrogène.

13. Dérivé tel que revendiqué dans la revendication 8, dans lequel m' vaut 19, B'₁ à B'₂₁ représentent, en séquence, G, U, C, C, C, U, G, U, U, C, G, G, G, C, G, C, C, A, C, U et G, Q'₁ à Q'₂₀ représentent un anion thio, Y'₁ et Y'₂ représentent OCH₃, Y'₃ représente OH, et R' représente un atome d'hydrogène.

14. Dérivé tel que revendiqué dans la revendication 8, dans lequel m' vaut 19, B'₁ à B'₂₁ représentent, en séquence, G, A, G, C, U, C, C, C, A, G, G, C, U, C, A, G, A, U, C, U et G, Q'₁ à Q'₂₀ représentent un anion thio, Y'₁ et Y'₂ représentent OCH₃, Y'₃ représente OH, et R' représente un atome d'hydrogène.

15. Dérivé tel que revendiqué dans la revendication 8, dans lequel m' vaut 18, B'₁ à B'₂₀ représentent, en séquence, A, C, A, C, C, C, A, A, U, U, C, U, G, A, A, A, A, U, G et G, Q'₁ à Q'₅ représentent un anion thio, Q'₆ à Q'₁₄ représentent un anion oxo, au moins 2 parmi Q'₁₅ à Q'₁₉ représentent un anion thio et les 3 autres représentent chacun un anion oxo, Y'₁ et Y'₂ représentent OCH₃, Y'₃ représente OH, et R' représente un atome d'hydrogène.

16. Dérivé de 2'-O-méthylribooligonucléotide phosphorothioate, représenté par la formule générale : 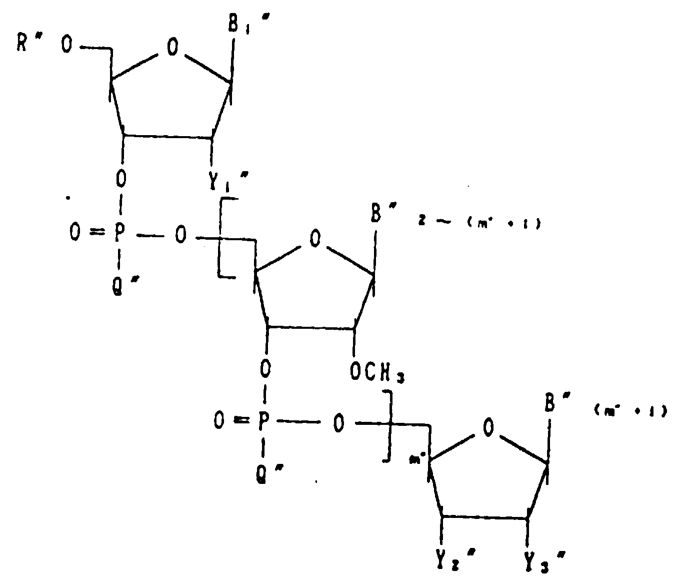 dans laquelle :
m" est un nombre entier compris entre 1 et 50 ;
B"₁ à B"_{(m"+2)}, qui peuvent être identiques ou différents, représentent chacun l'un quelconque parmi l'adénine-9-yle, la guanine-9-yle, la cytosine-1-yle, l'uracil-1-yle, la thymine-1-yle et l'hypoxanthine-9-yle ;
Q" contient au moins un anion thio et représente l'un quelconque parmi un anion thio, un anion oxo, un groupe aryloxy, un groupe alkylamino, un groupe arylamino, un groupe alkylthio et un groupe arylthio;
Y"₁ à Y"₃ représentent indépendamment un groupe méthoxy, un groupe alkyloxy qui peut éventuellement porter un substituant, un groupe hydroxy, un groupe alkylsilyle et un atome d'hydrogène ;
R" représente un atome d'hydrogène ou un groupe phosphoryle qui peut éventuellement porter un substituant.

17. Dérivé tel que revendiqué dans la revendication 16, dans lequel m" vaut 18, B"₁ à B"₂₀ représentent une adénine-9-yle, Q" représente un anion thio, Y"₁ à Y"₂ représentent OCH₃, Y"₃ représente OH, et R" représente un atome d'hydrogène.

18. Dérivé tel que revendiqué dans la revendication 16, dans lequel m" vaut 18, B"₁ à B"₂₀ représentent une hypoxanthine-9-yle, Q' représente un anion thio, Y"₁ à Y"₂ représentent OCH₃, Y"₃ représente OH, et R" représente un atome d'hydrogène.

19. Dérivé tel que revendiqué dans la revendication 16, dans lequel m" représente 18, B"₁ à B"₂₀ représentent un uracil-1-yle, Q" représente un anion thio, Y"₁ à Y"₂ représentent OCH₃, Y"₃ représente OH, et R" représente un atome d'hydrogène.

20. Dérivé tel que revendiqué dans la revendication 16, dans lequel m" vaut 18, B"₁ à B"₂₀ représentent une cytosine-1-yle, Q" représente un anion thio, Y"₁ à Y"₂ représentent OCH₃, Y"₃ représente OH, et R" représente un atome d'hydrogène.

21. Dérivé d'oligoribonucléotide représenté par la formule générale : 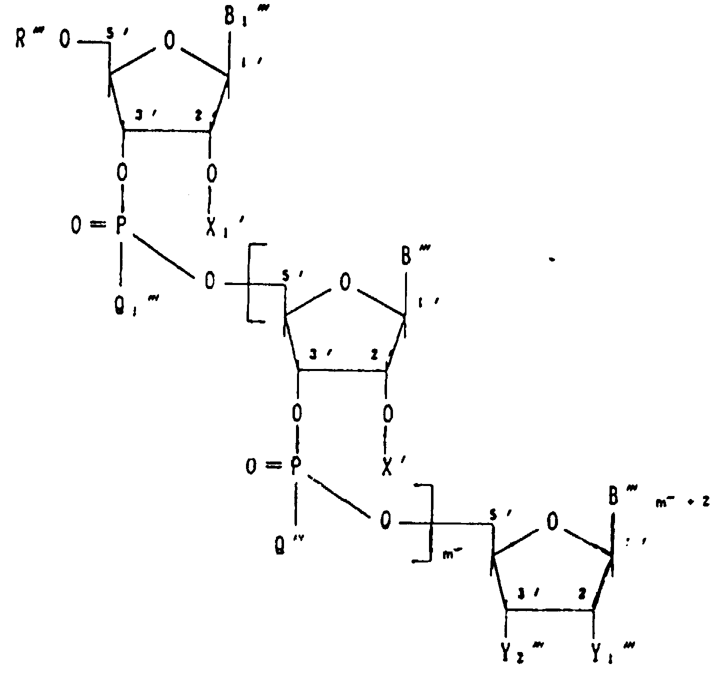 dans laquelle :
m"' est un nombre entier compris entre 1 et 100 ;
B"' représente B"'₂, B"'₃, ..... B"'_{(m"'+1)}, et B"'₁ à B"'_{(m"'+2)}, qui peuvent être identiques ou différents, représentent chacun l'un quelconque parmi l'hypoxanthine-9-yle, la guanine-9-yle, l'adénine-9-yle, la cytosine-1-yle, l'uracil-1-yle et la thymine-1-yle à la condition que tous ne représentent pas à la fois l'adénine-9-yle ;
Q"' représente Q"'₂, Q"'₃,.....Q"'_{(m"'+1)}, et Q"'₁ à Q"'_{(m"'+1)}, représentent indépendamment l'un quelconque parmi un anion thio, un anion oxo, un groupe aryloxy, un groupe alkylamino, un groupe arylamino, un groupe alkylthio et un groupe arylthio (à condition qu'au moins l'un parmi Q"'₁ à Q"'_{(m"+1)} représente un anion thio);
X' représente X'₂, X'₃ ..... X'_{(m"'+1)} et, X'₁ à X'_{(m"'+1)}, représentent chacun un atome d'hydrogène ou un groupe méthyle, (à condition que tous ne représentent pas en même temps un groupe méthyle, et à condition que tous ne représente pas ensemble un atome d'hydrogène,
R"' représente l'un quelconque parmi un atome d'hydrogène, un groupe acyle comportant de 1 à 20 atomes de carbone, un groupe phosphoryle qui peut éventuellement porter un substituant, et
Y"'₁ et Y"'₂ représentent indépendamment l'un quelconque parmi un groupe méthoxy, un groupe acyloxy comportant de 1 à 20 atomes de carbone, un groupe alkyloxy qui peut éventuellement porter un substituant, un groupe hydroxyle, un groupe alkylsilyle, un atome d'hydrogène et un groupe phosphoryle qui peut éventuellement porter un substituant.

22. Dérivé tel que revendiqué dans la revendication 21, dans lequel ledit substituant est l'un quelconque parmi un groupe cyanoéthyle, un groupe chlorophényle et un résidu hydrocarboné comportant de 1 à 20 atomes de carbone.

23. Dérivé tel que revendiqué dans la revendication 21, ayant la formule de structure :
